# EUROPEAN PATENT SPECIFICATION

(11) **EP 4 182 303 B1**
(45) Date of publication and mention of the grant of the patent: **29.11.2023**
(21) Application number: 22748028.2
(22) Date of filing: 11.07.2022
(51) Int. Cl.: C07D 257/04, A61P 35/00, A61K 31/41

(54) **THERMODYNAMICALLY STABLE FORM OF SCO-101**
THERMODYNAMISCH STABILE FORM VON SCO-101
FORME THERMODYNAMIQUEMENT STABLE DE SCO-101

(30) Priority: 16.07.2021 EP 21186075
(43) Date of publication of application: 24.05.2023
(73) Proprietor: Scandion Oncology A/S, 2100 Copenhagen Ø (DK)
(72) Inventor: RASMUSSEN, Kaare, G., 2100 Copenhagen (DK); EGEBRO, René, 2100 Copenhagen (DK); JENSEN, Klaus, Snej, 1920 Frederiksberg (DK); LOBATO, Kiara, Glasgow Aberdeenshire G20 8PD (GB); SHARP, Lorraine, Edinburgh Aberdeenshire EH14 1PL (GB)
(74) Representative: IPTector Consulting ApS
(86) International application number: PCT/EP2022/069240
(87) International publication number: WO 2023/285344

(56) References cited:
- WO-A1-00/24707

## Description

### Technical field

The present disclosure relates to an improved crystal form of SCO-101, its preparation and use. Further, the disclosure relates to intermediary crystal forms of SCO-101 that can be converted to the improved crystal form of SCO-101.

### Background

Cancer is an overwhelming burden to our society with approximately 18 million new cases of cancer diagnosed in 2019. Despite the introduction of many new treatment modalities/options, *de novo* or acquired resistance to the applied treatments still represents the major cause of death from cancer.

The compound SCO-101, also known as NS3728 was first described in WO 2000/24707. SCO-101 has later been shown to be an effective potentiator of a range of anti-cancer agents and is currently being developed for cancer combination therapies, in particular for treatment of resistant cancers. WO 2017/198700 describes SCO-101 and its use in combination therapies for treatment of cancers.

A range of substituted diphenyl ureas including SCO-101 can be prepared as described in WO 2000/24707 by mixing suitable starting materials soluble in toluene whereby SCO-101 precipitates when formed. SCO-101 prepared in toluene using the conditions of WO 2000/24707 is crystalline.

### Summary

The present inventors have found that the polymorphic form of SCO-101 prepared as in WO 2000/24707 is not a thermodynamically stable form but rather a metastable polymorphic form which is hygroscopic. The polymorphic form of SCO-101 prepared as in WO 2000/24707 is referred to herein as crystal form II. Further, SCO-101 has been shown to readily form solvates and several polymorphs exist. Thus, from a pharmaceutical development and patient safety point of view, it is desired to obtain a more stable crystal form, free of solvates (anhydrous). Further, it is desired to provide metastable forms that can be converted into the desired thermodynamically stable form, referred to as crystal form I herein. The present inventors have further demonstrated that the thermodynamically stable form, crystal form I, unexpectedly is non-hygroscopic, rendering crystal form I of SCO-101 a more attractive crystal form for clinical development.

In a first aspect, crystal form I of SCO-101 is provided: 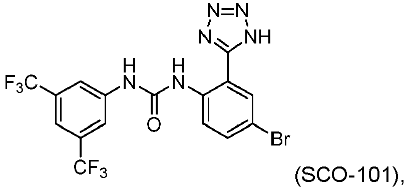 exhibiting at least peak maxima at 2 Theta angles: 19.0±0.2, 21.2±0.2, and 23.4±0.2 in an X-ray powder diffraction (XRPD) diffractogram when measured using Cu K_{α} radiation; optionally wherein the crystal form I exhibits at least peak maxima at 2 Theta angles: 13.9±0.2, 19.0±0.2, 19.9±0.2, and 21.2±0.2 in an X-ray powder diffraction (XRPD) diffractogram when measured using Cu K_{α} radiation, for example wherein the crystal form I exhibits at least peak maxima at 2 Theta angles: 12.0±0.2, 13.9±0.2, 19.0±0.2, 19.9±0.2, 20.4±0.2, 21.2±0.2, 23.2±0.2, 23.4±0.2, 26.9±0.2, and 27.4±0.2 in an X-ray powder diffraction (XRPD) diffractogram when measured using Cu K_{α} radiation.

In a second aspect, a crystal form III of SCO-101 is provided: 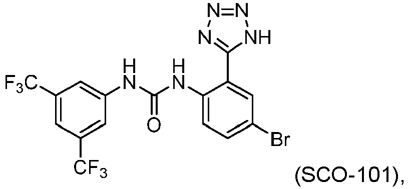 exhibiting at least peak maxima at 2 Theta angles: 11.1 ±0.2, 21.7±0.2, and 23.3±0.2 in an X-ray powder diffraction (XRPD) diffractogram when measured using Cu K_{α} radiation, optionally wherein the crystal form III exhibits at least peak maxima at 2 Theta angles: 11.1±0.2, 21.7±0.2, 23.3±0.2, and 26.2±0.2 in an X-ray powder diffraction (XRPD) diffractogram when measured using Cu K_{α} radiation, for example wherein the crystal form III exhibits at least peak maxima at 2 Theta angles: 11.1±0.2, 16.6±0.2, 18.0±0.2,19.2±0.2, 19.9±0.2, 21.7±0.2, 22.2±0.2, 22.5±0.2, 23.3±0.2, and 26.2±0.2 in an X-ray powder diffraction (XRPD) diffractogram when measured using Cu K_{α} radiation.

In a third aspect, a crystal form IV of SCO-101 is provided: 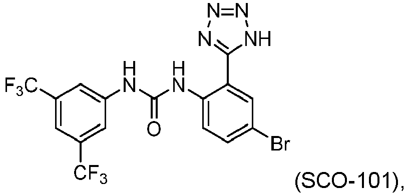 exhibiting at least peak maxima at 2 Theta angles: 22.6±0.2, 23.4±0.2, and 23.7±0.2 in an X-ray powder diffraction (XRPD) diffractogram when measured using Cu K_{α} radiation, optionally wherein the crystal form IV exhibits at least peak maxima at 2 Theta angles: 21.6±0.2, 22.6±0.2, 23.4±0.2, 23.7±0.2, and 24.1±0.2 in an X-ray powder diffraction (XRPD) diffractogram when measured using Cu K_{α} radiation, for example wherein the crystal form IV exhibits at least peak maxima at 2 Theta angles: 13.5±0.2, 20.1 ±0.2, 21.6±0.2, 22.6±0.2, 23.4±0.2, 23.7±0.2, 24.1±0.2, 25.6±0.2, 27.4±0.2, and 30.2±0.2 in an X-ray powder diffraction (XRPD) diffractogram when measured using Cu K_{α} radiation.

In a fourth aspect, an amorphous form of SCO-101 is provided: 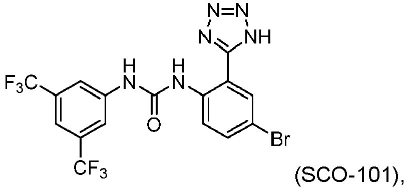 exhibiting no peak maxima at 2 Theta angles between 0 and 40 in an X-ray powder diffraction (XRPD) diffractogram when measured using Cu K_{α} radiation.

In a fifth aspect, a crystal form V of SCO-101 isopropanol solvate is provided: 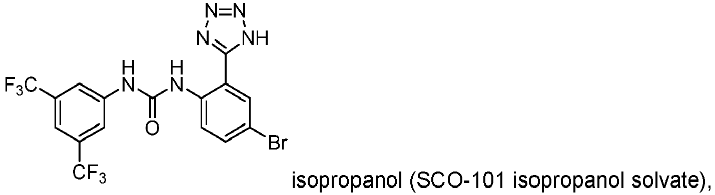 exhibiting at least peak maxima at 2 Theta angles: 9.4±0.2, 21.1 ±0.2, and 22.2±0.2 in an X-ray powder diffraction (XRPD) diffractogram when measured using Cu K_{α} radiation, optionally wherein the crystal form V exhibits at least peak maxima at 2 Theta angles: 8.2±0.2, 9.4±0.2, 21.1±0.2, and 22.2±0.2 in an X-ray powder diffraction (XRPD) diffractogram when measured using Cu K_{α} radiation; for example wherein the crystal form V exhibits at least peak maxima at 2 Theta angles: 8.2±0.2, 9.4±0.2, 10.5±0.2, 21.1±0.2, 22.2±0.2, 23.7±0.2, 24.2±0.2, 24.6±0.2, 25.5±0.2, and 28.5±0.2 in an X-ray powder diffraction (XRPD) diffractogram when measured using Cu K_{α} radiation.

In a sixth aspect, a process is provided for preparing a crystal form I of SCO-101 as defined herein, the process comprising the consecutive steps of:
a) dissolving SCO-101 in one or more polar aprotic solvents selected from the group consisting of: acetone, acetonitrile, dichloromethane, dimethylformamide, dimethylpropyleneurea, dimethylsulfoxide, ethyl acetate, 2-MeTHF, and tetrahydrofuran, for example wherein the polar aprotic solvent is acetone, at a first predefined temperature from 0 to 20 °C, such as 10 ± 5 °C;
b) adding one or more polar protic solvents selected from the group consisting of: water, methanol, ethanol, isopropanol, and acetic acid, for example wherein the polar protic solvent is water to the one or more polar aprotic solvents over the course of a first predefined time period from 10 to 360 minutes, such as from 70 to 90 minutes to provide the crystal form I of SCO-101; and
c) isolating the crystal form I of SCO-101.

In a seventh aspect, a process is provided for preparing the crystal form I of SCO-101 as defined herein from a metastable form, comprising:
a) providing a metastable form which is a crystal form or an amorphous form of SCO-101;
b) mixing the metastable form with the crystal form I of SCO-101 as defined herein in a solvent mixture of: i) one or more polar aprotic solvents selected from the group consisting of: acetone, acetonitrile, dichloromethane, dimethylformamide, dimethylpropyleneurea, dimethylsulfoxide, ethyl acetate, 2-MeTHF, and tetrahydrofuran, and
ii) one or more polar protic solvents selected from the group consisting of: water, methanol, ethanol, isopropanol, and acetic acid, or one or more apolar solvents selected from the group consisting of: heptane, hexane, pentane, cyclohexane, toluene, and diethyl ether; for example wherein the polar aprotic solvent is acetone and the polar protic solvent is water;
c) agitating the solvent mixture at from 30 to 60 °C for at least 1 hour thereby providing the crystal form I of SCO-101;
wherein the crystal form of SCO-101 is selected from the group consisting of:
   i) a crystal form II of SCO-101 : 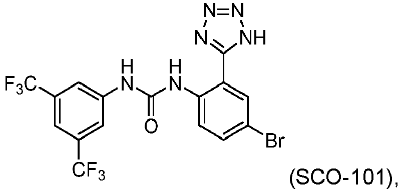 exhibiting at least peak maxima at 2 Theta angles: 18.8±0.2, 23.2±0.2, and 20.5±0.2 in an X-ray powder diffraction (XRPD) diffractogram when measured using Cu K_{α} radiation;
   ii) a crystal form III of SCO-101 as defined herein;
   iii) a crystal form IV of SCO-101 as defined herein; and
   iv) a crystal form V of SCO-101 as defined herein, and
wherein the amorphous form of SCO-101 is as defined herein.

In an eighth aspect, a pharmaceutical composition is provided comprising a crystal form I of SCO-101 as defined herein; and one or more pharmaceutically acceptable adjuvants, excipients, carriers, buffers and/or diluents.

In a ninth aspect, the present disclosure provides a crystal form I of SCO-101 as defined herein, or a pharmaceutical composition as defined herein for use in the treatment of cancer in combination with one or more anti-cancer agents; optionally wherein the one or more anti-cancer agents are selected from the group consisting of topoisomerase inhibitors, anti-hormone agents, alkylating agents, mitotic inhibitors, antimetabolites, anti-tumor antibiotics, corticosteroids, targeted anti-cancer therapy, differentiating agents and immunotherapy.

### Description of Drawings

**Fig. 1A****:** This figure shows the XRPD diffractogram of polymorph Form I of the compound *N*-[4-Bromo-2-(1*H*-1,2,3,4-tetrazol-5-yl)phenyl]-*N*'-[3,5-bis(trifluoromethyl)phenyl]urea (SCO-101).
**Fig. 1B****:** This figure shows the XRPD diffractogram of polymorph Form II of the compound *N*-[4-Bromo-2-(1H-1,2,3,4-tetrazol-5-yl)phenyl]-*N*'-[3,5-bis(trifluoromethyl)phenyl]urea (SCO-101).
**Fig. 1C****:** This figure shows the XRPD diffractogram of polymorph Form III of the compound *N*-[4-Bromo-2-(1*H*-1,2,3,4-tetrazol-5-yl)phenyl]-*N*'-[3,5-bis(trifluoromethyl)phenyl]urea (SCO-101).
**Fig. 1D****:** This figure shows the XRPD diffractogram of polymorph Form IV of the compound *N*-[4-Bromo-2-(1*H*-1,2,3,4-tetrazol-5-yl)phenyl]-*N*'-[3,5-bis(trifluoromethyl)phenyl]urea (SCO-101).
**Fig. 1E****:** This figure shows the XRPD diffractogram of the amorphous form of the compound *N*-[4-Bromo-2-(1*H*-1,2,3,4-tetrazol-5-yl)phenyl]-*N*'-[3,5-bis(trifluoromethyl)phenyl]urea (SCO-101). No 2-theta peaks are observed for an amorphous compound.
**Fig. 1F****:** This figure shows the XRPD diffractogram of the 2-propanol solvate of the compound *N*-[4-Bromo-2-(1*H*-1,2,3,4-tetrazol-5-yl)phenyl]-*N*'-[3,5-bis(trifluoromethyl)phenyl]urea (SCO-101) referred to as Form V or Pattern 5, used equivalently.

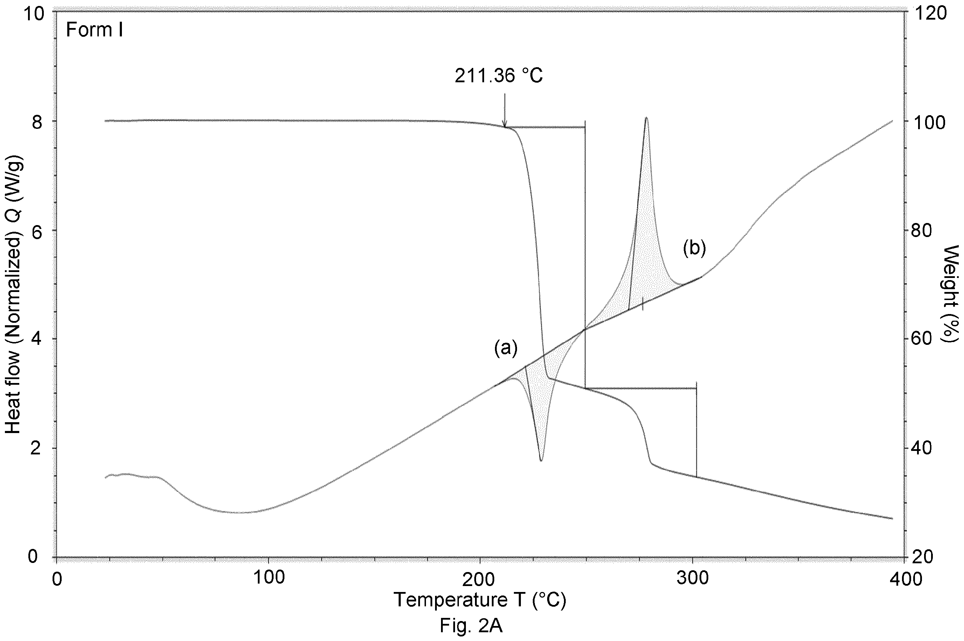

**Fig. 2A****:** This figure shows the TG/DSC thermogram of polymorph Form I of the compound N-[4-Bromo-2-(1H-1,2,3,4-tetrazol-5-yl)phenyl]-N'-[3,5-bis(trifluoromethyl)phenyl]urea (SCO-101). Two stepped mass losses were observed in the TG trace, a 48 wt. % mass loss (C9H7F6N) between 211 and 250 °C and a 16 wt. % (CH2N4) mass loss between 250 and 300 °C. As no further mass losses were observed it is concluded that Form I is a non-solvated and anhydrous polymorph. In the DSC trace, an endothermic event with an onset at 221 °C (melting point) was observed concurrent with the 48 wt. % mass loss, this was followed by an exothermic degradation event with an onset at 270 °C concurrent with the 16 wt. % mass loss. (a) Enthalpy (normalized): 121.68 J/g; Peak temperature: 228.92 °C; Onset temperature: 221.30 °C. (b) Enthalpy (normalized): 238.68 J/g; Peak temperature: 278.42 °C; Onset temperature: 269.84 °C.
**Fig. 2B****:** This figure shows the TG/DSC thermogram of polymorph Form II of the compound N-[4-Bromo-2-(1H-1,2,3,4-tetrazol-5-yl)phenyl]-N'-[3,5-bis(trifluoromethyl)phenyl]urea (SCO-101). Two stepped mass losses were observed in the TG trace, a 54 wt. % mass loss between 189 and 248 °C and a 20 wt. % mass loss between 248 and 300 °C. As no further mass losses were observed it is concluded that Form II is a non-solvated and anhydrous polymorph.
   In the DSC trace, an endothermic event with an onset at 214 °C (melting point) was observed concurrent with the 54 wt. % mass loss, this was followed by an exothermic degradation event with an onset at 273 °C concurrent with the 20 wt. % mass loss. (a) Enthalpy (normalized): 67.701 J/g; Peak temperature: 222.81 °C; Onset temperature: 213.99 °C. (b) Enthalpy (normalized): 333.24 J/g; Peak temperature: 283.20 °C; Onset temperature: 272.94 °C.

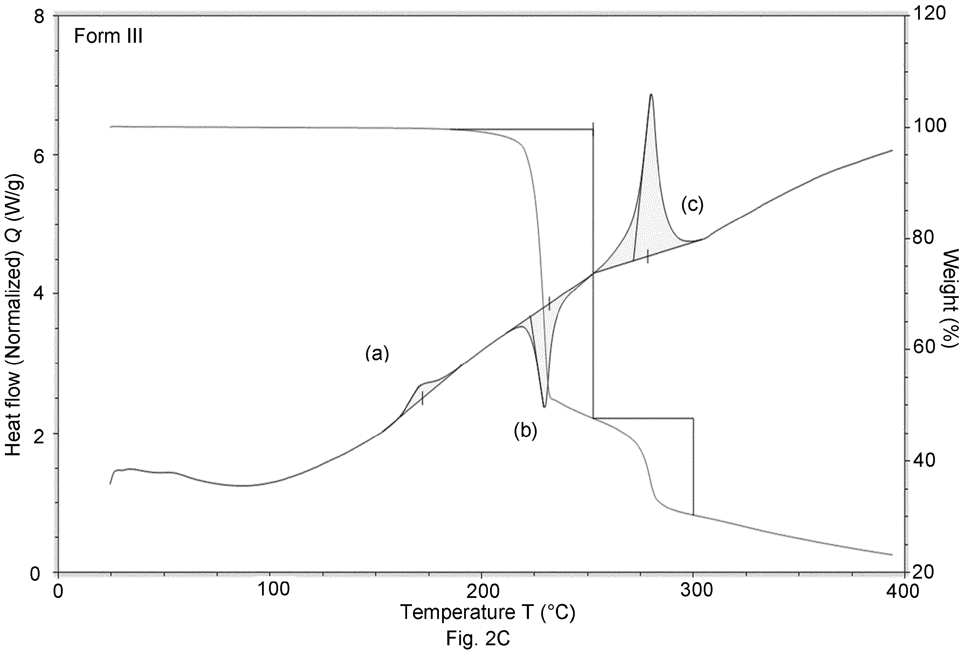

**Fig. 2C****:** This figure shows the TG/DSC thermogram of polymorph Form III of the compound N-[4-Bromo-2-(1H-1,2,3,4-tetrazol-5-yl)phenyl]-N'-[3,5-bis(trifluoromethyl)phenyl]urea (SCO-101). In the TG trace, two stepped mass losses due to decomposition of the API were observed of 52.0 wt. % between 185 and 250 °C, and 17.4 wt. % between 250 and 300 °C. No additional mass losses were observed. In the DSC trace, one additional exothermic event was observed that was not present for form I with an onset at 162 °C. Further analysis by VT-XRPD (variable temperature XRPD) has shown that this exothermic event is caused by the conversion of Form III into Form IV showing a mixture of Form III and Form IV at 150°C and the complete transformation into Form IV at 155° C. This was followed by an endothermic event with an onset at 223 °C (melting point) followed by an exothermic event with an onset at 272 °C due to decomposition of the API. (a) Enthalpy (normalized): 15.517 J/g; Peak temperature: 171.12 °C; Onset temperature: 161.86 °C. (b) Enthalpy (normalized): 71.642 J/g; Peak temperature: 229.90 °C; Onset temperature: 223.10 °C. (c) Enthalpy (normalized): 162.97 J/g; Peak temperature: 280.15 °C; Onset temperature: 271.68 °C.

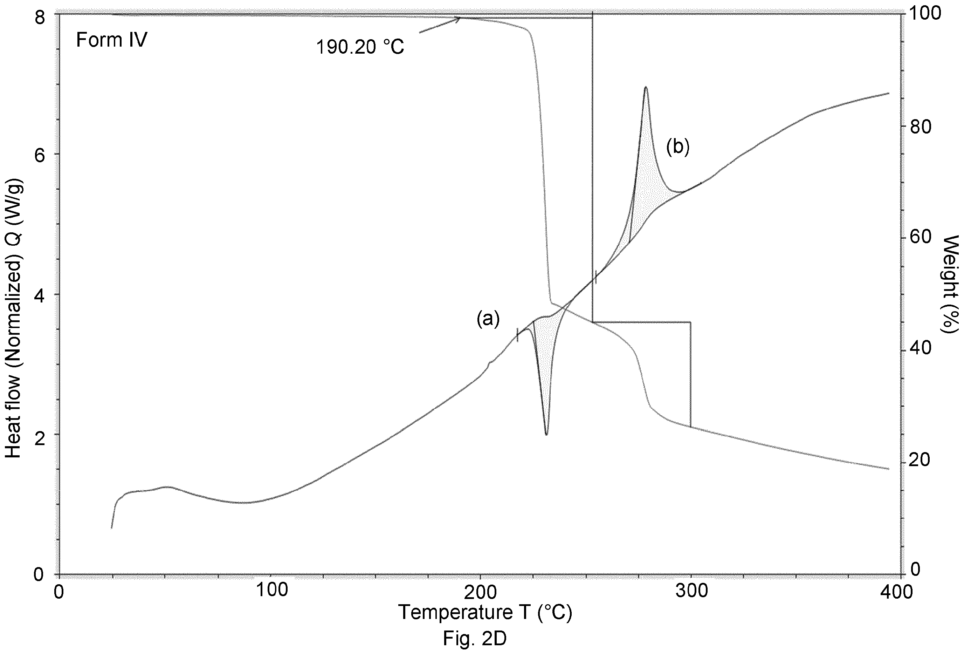

**Fig. 2D****:** This figure shows the TG/DSC thermogram of polymorph Form IV of the compound N-[4-Bromo-2-(1H-1,2,3,4-tetrazol-5-yl)phenyl]-N'-[3,5-bis(trifluoromethyl)phenyl]urea (SCO-101). In the TG trace, No mass losses were observed until the two stepped mass losses due to decomposition of the API. These were 54.3 wt. % between 190 and 255 °C, and 16.7 wt. % between 255 and 300 °C. As no further mass losses were observed it is concluded that Form IV is a non-solvated and anhydrous polymorph. An endothermic event was observed with an onset at 225 °C followed by an exothermic event with an onset at 271 °C due to decomposition of the API. (a) Enthalpy (normalized): 68.722 J/g; Peak temperature: 231.40 °C; Onset temperature: 225.20 °C. (b) Enthalpy (normalized): 116.26 J/g; Peak temperature: 278.52 °C; Onset temperature: 270.65 °C.

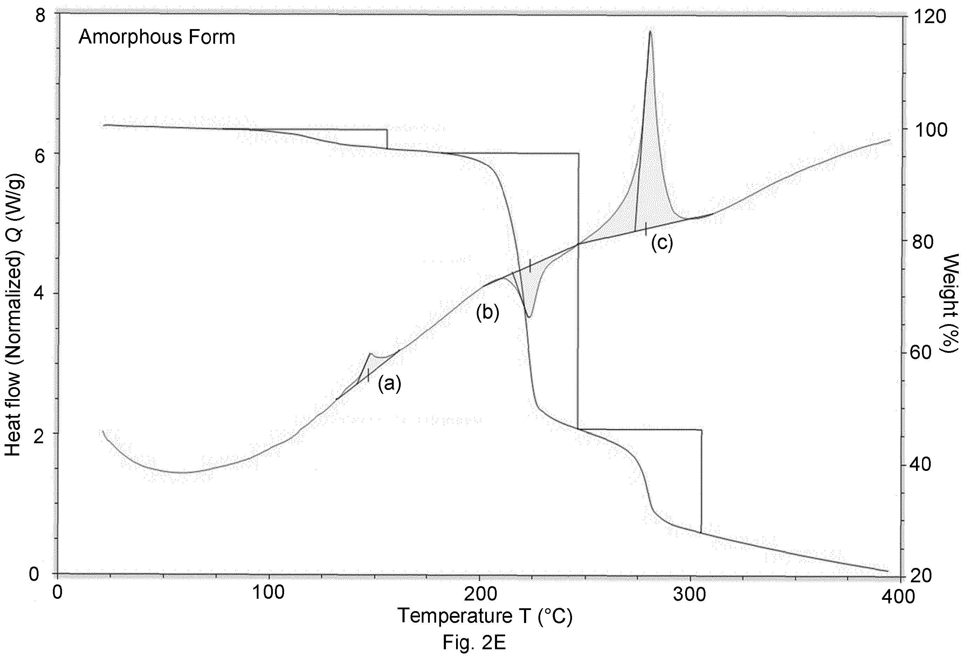

**Fig. 2E****:** This figure shows the TG/DSC thermogram of the amorphous form of the compound *N*-[4-Bromo-2-(1*H*-1,2,3,4-tetrazol-5-yl)phenyl]-*N'*-[3,5-bis(trifluoromethyl)phenyl]urea (SCO-101). In the TG trace, a 3.4 wt. % (0.3 eq. acetone or 0.97 eq. water) mass loss was observed between 90 and 155 °C. This was followed by two stepped mass losses due to decomposition of the API of 49 wt. % between 180 and 247 °C, and 18 wt. % between 247 and 305 °C. In the DSC trace, a small exothermic event was observed with an onset at 141 °C concurrent with the acetone mass loss. Additionally, an endothermic event with an onset at 215 °C followed by an exothermic event with an onset at 273 °C were observed concurrent with the decomposition mass losses. (a) Enthalpy (normalized): 16.389 J/g; Peak temperature: 147.62 °C; Onset temperature: 141.31 °C. (b) Enthalpy (normalized): 46.123 J/g; Peak temperature: 223.34 °C; Onset temperature: 215.33 °C. (c) Enthalpy (normalized): 192.48 J/g; Peak temperature: 280.08 °C; Onset temperature: 273.03 °C.

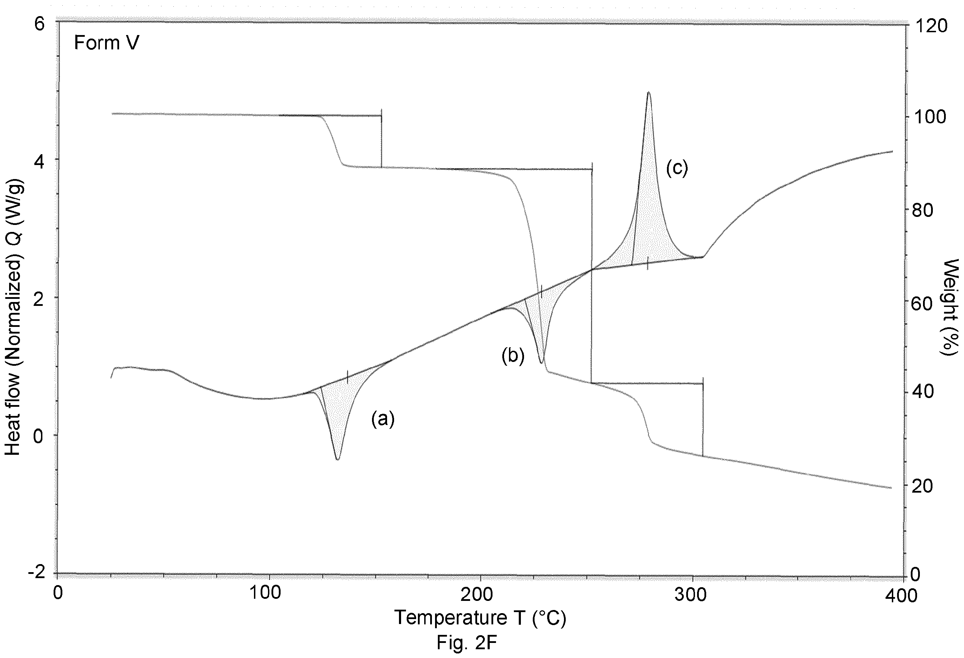

**Fig. 2F****:** This figure shows the TG/DSC thermogram of the 2-propanol (isopropanol) solvate form of the compound N-[4-Bromo-2-(1H-1,2,3,4-tetrazol-5-yl)phenyl]-N -[3,5-bis(trifluoromethyl)phenyl]urea (SCO-101) i.e. Form V. In the TG trace, one stepped mass loss was observed that was not present in the received material. An 11.2 wt. % (1.0 eq. 2-propanol or 3.5 eq. water) mass loss between 100 and 150 °C. This was followed by two stepped mass losses due to decomposition of the API of 46.6 wt. % between 180 and 250 °C, and 15.6 wt. % between 250 and 305 °C. In the DSC trace, one additional endothermic event was observed that was not present for Form I with an onset at 124 °C concurrent with the 2-propanol mass loss. This was followed by an endothermic event with an onset at 220 °C followed by an exothermic event with an onset at 271 °C due to decomposition of the API. These data support that the present Form V is an isopropanol solvate. (a) Enthalpy (normalized): 81.609 J/g; Peak temperature: 132.09 °C; Onset temperature: 123.79 °C. (b) Enthalpy (normalized): 67.328 J/g; Peak temperature: 228.55 °C; Onset temperature: 220.37 °C. (c) Enthalpy (normalized): 175.03 J/g; Peak temperature: 278.52 °C; Onset temperature: 270.72 °C.

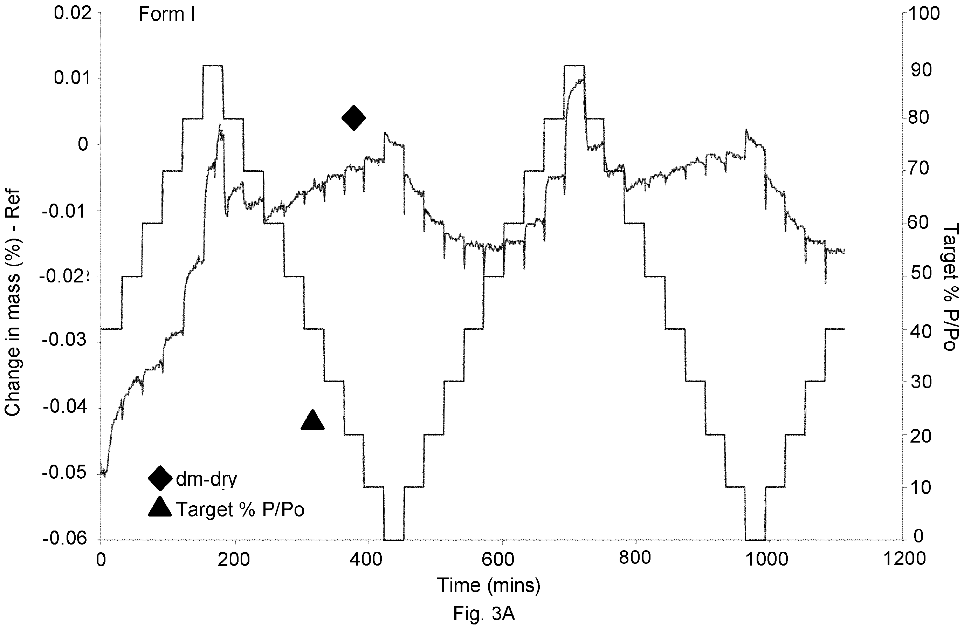

**Fig. 3A****:** This figure shows the DVS (Dynamic Vapor Sorption) diagram of polymorph Form I of the compound *N*-[4-Bromo-2-(1*H*-1,2,3,4-tetrazol-5-yl)phenyl]-*N*'-[3,5-bis(trifluoromethyl)phenyl]urea (SCO-101). The present figure demonstrates that Form I is non-hygroscopic as no increase in mass of a sample of Form I is observed when increasing the relative humidity to 90%.

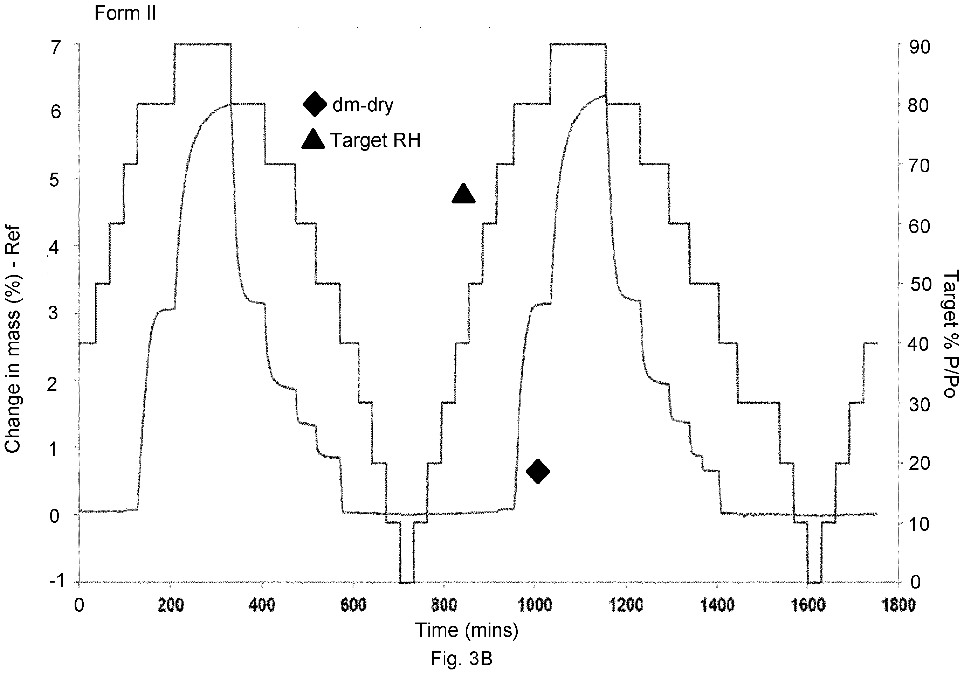

**Fig. 3B****:** This figure shows the DVS diagram of polymorph Form II of the compound *N*-[4-Bromo-2-(1*H*-1,2,3,4-tetrazol-5-yl)phenyl]-*N*'-[3,5-bis(trifluoromethyl)phenyl]urea (SCO-101). An increase of approx 6% in mass gain is observed at 90% relative humidity. The 3% weight gain when increasing RH from 70% to 80% corresponds to the formation of a monohydrate and the 6% weight gain in total corresponds to a dihydrate. The present figure demonstrates that Form II is hygroscopic.

### Detailed description

The present invention is defined by the appended claims. Any embodiment not falling under the scope of the appended claims does not form part of the invention.

### Definitions

The term "polymorph" or "polymorphic form" used herein refers to a polymorphic form of SCO-101. Solids exist in either amorphous or crystalline forms also referred to as crystal forms herein. In the case of crystal forms, the crystal's molecules are positioned in 3-dimensional lattice sites. When a compound recrystallizes from a solution or slurry, it may crystallize with different spatial lattice arrangements, a property referred to as "polymorphism," with the different crystal forms individually being referred to as a "polymorph". Different polymorphic forms of a given substance may differ from each other with respect to one or more physical properties, such as solubility and dissociation, true density, crystal shape, compaction behavior, flow properties, and/or solid state stability. In the case of a chemical substance that exists in two (or more) polymorphic forms, the unstable forms generally convert to the more thermodynamically stable forms at a given temperature after a sufficient period of time. When this transformation is not rapid, the thermodynamically unstable form is referred to as the "metastable" form.

Unless otherwise specified, the unit of 2 Theta angles is degrees (°). When the 2 Theta angles are used to characterize a polymorph's distinctive XRPD peak maxima, each 2 Theta angle includes ±0.2° to account for error margins. Thus, a XRPD peak maximum defined by 2 Theta angle 10.0 will include 9.8 and 10.2, and any number in between, represented by 10.0±0.2.

The term "onset temperature" used herein refers to the designed intersection point of the extrapolated baseline and the inflectional tangent at the beginning of the melting in a DSC/TG experiment.

The term "peak temperature" used herein refers to a local maximum or minimum temperature which can be determined by means of differential scanning calorimetry (DSC), which is known to one skilled in the art.

The term "seeding" used herein refers to the technique of adding one or more "seed" crystals to the crystallization solution to promote the formation of crystals.

The term "amorphous form" as used herein, refers to a non-crystalline form of a substance as determined by X-ray powder diffraction (XRPD). The term "amorphous" form cover solids of disordered arrangements of molecules and do not possess a distinguishable crystal lattice.

The term "polymorphic form" refers to the property of SCO-101 to exist in different crystal forms with distinct crystal lattices providing differences of the crystalline material in e.g. crystal hardness, shape and size. The different crystalline forms or crystal forms can be identified and examined by crystallographic techniques such as XRPD and DSC/TG or indirectly by assessment of differences in physical and/or chemical properties associated with each particular polymorph. The different polymorphs vary in physical properties such as solubility, dissolution, solid-state stability as well as processing behaviour in terms of powder flow and compaction during tabletting.

The term "anti-cancer agent" as used herein includes, but is not limited to, a chemotherapeutic agent, that has activity against a susceptible tumour.

The term "polar protic solvent" refers to a polar solvent that is capable of exchanging protons with the reagents and that contains a polarizable proton.

The term "polar aprotic solvent" refers to a polar solvent which does not contain acidic hydrogen and does not act as a hydrogen bond donor.

The term "apolar solvent" refers to a solvent which has a low dielectric constant (ε), preferably less than 9.5, and is not miscible with water.

The term "relative humidity" or "RH" refers to the ratio in percentage of the partial pressure of water vapor to the equilibrium vapor pressure of water at a given temperature.

The term "hygroscopic" is used herein to describe a compound or polymorphic form that sorbs water, either by absorption, adsorption, or a combination of the two processes.

The term "Form I" and "crystal form I" are used herein interchangeably. The same applies for Forms I, II, III, IV, and V.

### Crystal forms of SCO-101

### Crystal form I

SCO-101 Form I has been shown to be non-hygroscopic and the thermodynamically most stable polymorph of SCO-101. In addition, Form I has shown superior properties when compared to the other non-solvated forms and solvated forms of SCO-101 such as form II. Form I has a higher melting point than form II (Fig. 2A vs.

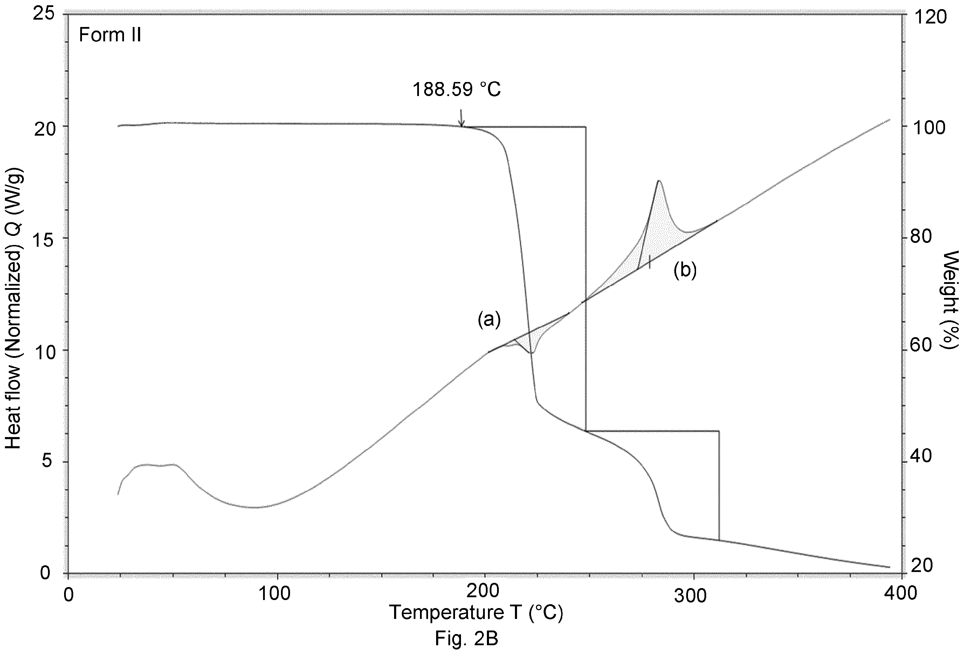

Fig 2B), and together with the results from the competitive slurry experiments (Example 7), it can be concluded that Form I is a more thermodynamically stable polymorph of SCO-101 compared to the other forms identified. Furthermore, DVS analysis of Form I (Fig. 3A) and Form II (Fig. 3B), has shown that Form I is essentially non-hygroscopic, whereas Form II absorbs approx. 6% water (w/w) at 90% RH. These experimental observations support that crystal form I of SCO-101 is the most attractive crystal form for clinical development from a stability and non-hygroscopicity point of view.

Thus in one embodiment of the present disclosure, a crystal form I of SCO-101 is provided: 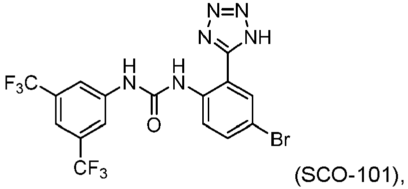 exhibiting at least peak maxima at 2 Theta angles: 19.0±0.2, 21.2±0.2, and 23.4±0.2 in an X-ray powder diffraction (XRPD) diffractogram when measured using Cu K_{α} radiation.

In one embodiment of the present disclosure, the crystal form I further exhibits one or more peak maxima at 2 Theta angles selected from the group consisting of: 13.9±0.2, 19.9±0.2, and 26.9±0.2 in an X-ray powder diffraction (XRPD) diffractogram when measured using Cu K_{α} radiation.

In one embodiment of the present disclosure, the crystal form I exhibits at least peak maxima at 2 Theta angles: 13.9±0.2, 19.0±0.2, 19.9±0.2, and 21.2±0.2 in an X-ray powder diffraction (XRPD) diffractogram when measured using Cu K_{α} radiation.

In one embodiment of the present disclosure, the crystal form I exhibits at least peak maxima at 2 Theta angles: 13.9±0.2, 19.0±0.2, 19.9±0.2, 21.2±0.2, and 23.4±0.2 in an X-ray powder diffraction (XRPD) diffractogram when measured using Cu K_{α} radiation.

In one embodiment of the present disclosure, the crystal form I exhibits at least peak maxima at 2 Theta angles: 13.9±0.2, 19.0±0.2, 19.9±0.2, 21.2±0.2, 23.4±0.2, and 26.9±0.2 in an X-ray powder diffraction (XRPD) diffractogram when measured using Cu K_{α} radiation.

In one embodiment of the present disclosure, the crystal form I exhibits at least peak maxima at 2 Theta angles: 12.0±0.2, 13.9±0.2, 19.0±0.2, 19.9±0.2, 21.2±0.2, 23.4±0.2, and 26.9±0.2 in an X-ray powder diffraction (XRPD) diffractogram when measured using Cu K_{α} radiation.

In one embodiment of the present disclosure, the crystal form I exhibits at least peak maxima at 2 Theta angles: 12.0±0.2, 13.9±0.2, 19.0±0.2, 19.9±0.2, 21.2±0.2, 23.4±0.2, 26.9±0.2, and 27.4±0.2 in an X-ray powder diffraction (XRPD) diffractogram when measured using Cu K_{α} radiation.

In one embodiment of the present disclosure, the crystal form I exhibits at least peak maxima at 2 Theta angles: 12.0±0.2, 13.9±0.2, 19.0±0.2, 19.9±0.2, 20.4±0.2, 21.2±0.2, 23.4±0.2, 26.9±0.2, and 27.4±0.2 in an X-ray powder diffraction (XRPD) diffractogram when measured using Cu K_{α} radiation.

In one embodiment of the present disclosure, the crystal form I exhibits at least peak maxima at 2 Theta angles: 12.0±0.2, 13.9±0.2, 19.0±0.2, 19.9±0.2, 20.4±0.2, 21.2±0.2, 23.2±0.2, 23.4±0.2, 26.9±0.2, and 27.4±0.2 in an X-ray powder diffraction (XRPD) diffractogram when measured using Cu K_{α} radiation.

In one embodiment of the present disclosure, the crystal form I exhibits at least peak maxima at 2 Theta angles according to table I in an X-ray powder diffraction (XRPD) diffractogram when measured using Cu K_{α} radiation.

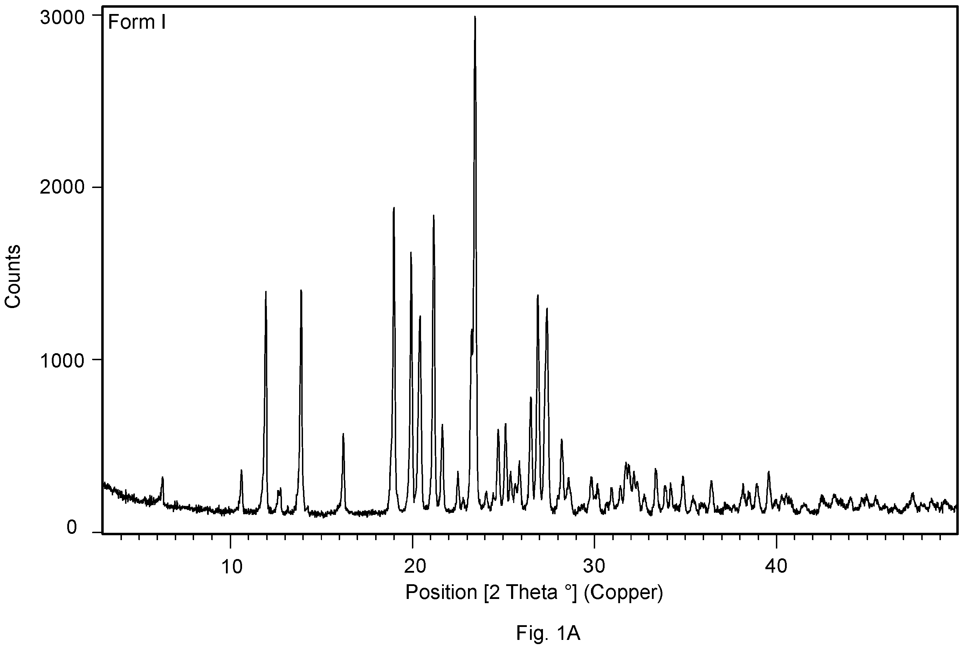

**Table I: The 2-theta values and intensity for the 20 most intense peaks of crystal form I, c.f. Fig. 1A.**

| **No.** | **Pos. [°2θ]** | **Rel. Int. [%]** |
|---|---|---|
| 1 | 11 .9585 | 41.71 |
| 2 | 13.8972 | 43.84 |
| 3 | 16.2329 | 15.37 |
| 4 | 18.9886 | 61.48 |
| 5 | 19.9395 | 51.88 |
| 6 | 20.4410 | 37.94 |
| 7 | 21.1652 | 60.04 |
| 8 | 21.6459 | 17.95 |
| 9 | 23.2446 | 36.41 |
| 10 | 23.4382 | 100.00 |
| 11 | 24.7179 | 16.44 |
| 12 | 25.1360 | 17.98 |
| 13 | 25.8837 | 10.20 |
| 14 | 26.5267 | 23.46 |
| 15 | 26.8950 | 43.10 |
| 16 | 27.4212 | 40.74 |
| 17 | 28.2188 | 14.94 |
| 18 | 31.6990 | 9.67 |
| 19 | 31.8933 | 8.83 |
| 20 | 33.3755 | 8.92 |

In one embodiment of the present disclosure, the crystal form I exhibits an XRPD diffractogram according to Fig 1A when measured using Cu K_{α} radiation.

### Crystal form I: Melting points (endothermic events)

In addition to XRPD diffractograms, the present polymorphs are also defined by their melting points. The melting points can be determined as an endothermic event observed by differential scanning calorimetry (DSC) which is not associated with a mass loss. The melting point is defined by either the onset temperature or the peak temperature of the endothermic event, or both.

In one embodiment of the present disclosure, the crystal form I exhibits in differential scanning calorimetry (DSC) an onset temperature of from 218 to 226 °C using a heating rate of 10 °C per minute, such as from 219 to 224 °C, such as from 220 to 222 °C, for example 221 °C.

In one embodiment of the present disclosure, the crystal form I exhibits in differential scanning calorimetry (DSC) a peak temperature of from 224 to 234 °C using a heating rate of 10 °C per minute, such as from 225 to 233 °C, such as from 226 to 232 °C, such as from 227 to 231 °C, such as from 228 to 230 °C, for example 229 °C. In one embodiment of the present disclosure, the crystal form I exhibits in differential scanning calorimetry (DSC) a peak temperature of 229 °C using a heating rate of 10 °C per minute.

### Crystal form II

The polymorphic form of SCO-101 prepared as in WO 2000/24707 is form II. The crystal form II of SCO-101: 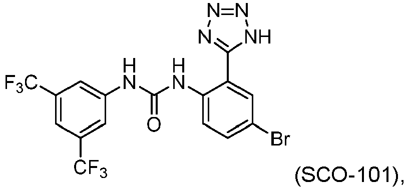 is characterized by exhibiting at least peak maxima at 2 Theta angles: 18.8±0.2, 23.2±0.2, and 20.5±0.2 in an X-ray powder diffraction (XRPD) diffractogram when measured using Cu K_{α} radiation. The crystal form II is a reference form not *per se* part of the present invention. The present invention is defined by the appended claims.

Crystal form II of SCO-101 can be identified using the 2-theta values presented in Table II.

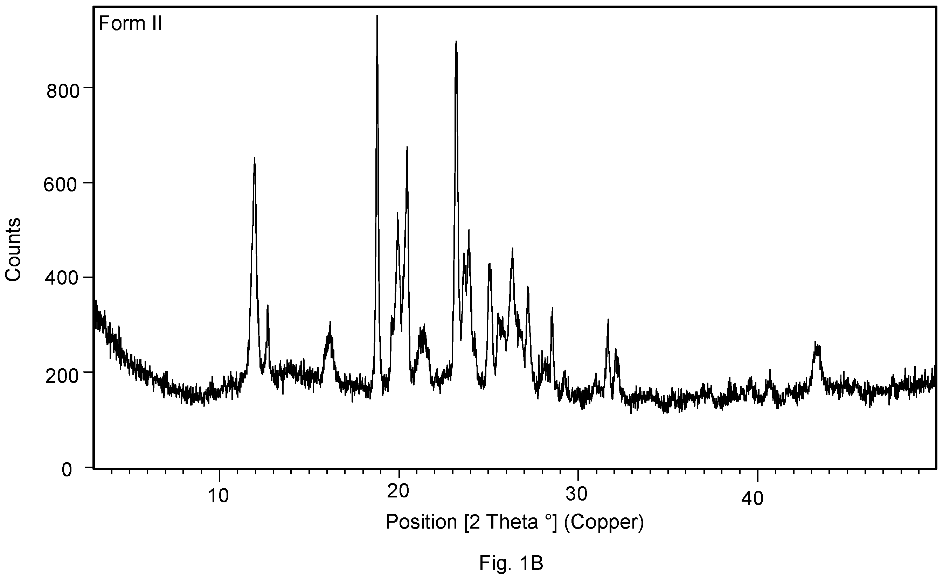

**Table II: The 2-theta values and intensity for the 20 most intense peaks of crystal form II, c.f. Fig. 1B.**

| **No.** | **Pos. [°2θ]** | **Rel. Int. [%]** |
|---|---|---|
| 1 | 12.0071 | 56.83 |
| 2 | 12.7014 | 18.74 |
| 3 | 16.1955 | 11.95 |
| 4 | 18.8106 | 100.00 |
| 5 | 19.5973 | 18.63 |
| 6 | 19.9321 | 42.47 |
| 7 | 20.4635 | 65.95 |
| 8 | 21.3370 | 13.82 |
| 9 | 23.1813 | 95.04 |
| 10 | 23.6239 | 35.30 |
| 11 | 23.9071 | 40.55 |
| 12 | 25.0309 | 34.40 |
| 13 | 25.6762 | 18.46 |
| 14 | 26.3107 | 35.20 |
| 15 | 27.2015 | 29.22 |
| 16 | 28.5399 | 21.01 |
| 17 | 29.2316 | 5.78 |
| 18 | 31.6454 | 19.55 |
| 19 | 32.0931 | 12.33 |
| 20 | 43.1779 | 10.14 |

### Crystal form III

A non-solvate crystal form of SCO-101 (Form III) can be obtained by temperature cycling of a slurry of amorphous SCO-101 in methanol as demonstrated by Example 5. The non-solvate crystal form of SCO-101 (Form III) can in turn be transformed to crystal form I as demonstrated by Example 7.

Thus in one embodiment of the present disclosure, a crystal form III of SCO-101 is provided: 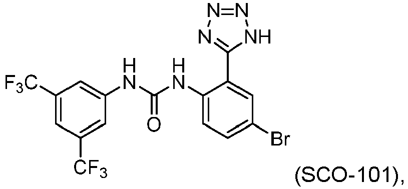 exhibiting at least peak maxima at 2 Theta angles: 11.1±0.2, 21.7±0.2, and 23.3±0.2 in an X-ray powder diffraction (XRPD) diffractogram when measured using Cu K_{α} radiation.

In one embodiment of the present disclosure, the crystal form III further exhibits one or more peak maxima at 2 Theta angles selected from the group consisting of: 19.9±0.2, 22.2±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 21.7±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 19.9±0.2, 21.7±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 19.9±0.2, 21.7±0.2, 22.2±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 16.6±0.2, 19.9±0.2, 21.7±0.2, 22.2±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 16.6±0.2, 19.9±0.2, 21.7±0.2, 22.2±0.2, 22.5±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 16.6±0.2, 19.2±0.2, 19.9±0.2, 21.7±0.2, 22.2±0.2, 22.5±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles: 11.1±0.2, 16.6±0.2, 18.0±0.2,19.2±0.2, 19.9±0.2, 21.7±0.2, 22.2±0.2, 22.5±0.2, 23.3±0.2, and 26.2±0.2 in an X-ray powder diffraction (XRPD) diffractogram when measured using Cu K_{α} radiation.

In one embodiment of the present disclosure, the crystal form III exhibits at least peak maxima at 2 Theta angles according to table III in an X-ray powder diffraction (XRPD) diffractogram when measured using Cu K_{α} radiation.

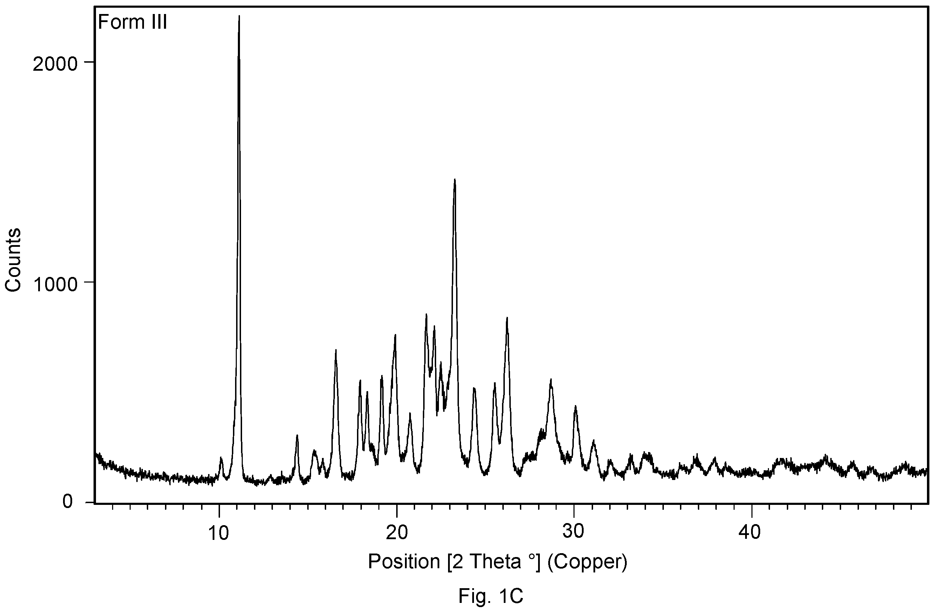

**Table III: The 2-theta values and intensity for the 20 most intense peaks of crystal form III, c.f. Fig. 1C.**

| **No.** | **Pos. [°2θ]** | **Rel. Int. [%]** |
|---|---|---|
| 1 | 11.1443 | 100.00 |
| 2 | 14.3752 | 10.77 |
| 3 | 15.3319 | 6.60 |
| 4 | 16.5742 | 29.71 |
| 5 | 17.9566 | 23.36 |
| 6 | 18.3639 | 20.78 |
| 7 | 19.1597 | 23.66 |
| 8 | 19.9219 | 31.97 |
| 9 | 20.7827 | 14.58 |
| 10 | 21.6544 | 37.34 |
| 11 | 22.1880 | 30.52 |
| 12 | 22.4986 | 25.21 |
| 13 | 23.2611 | 69.37 |
| 14 | 24.3506 | 20.96 |
| 15 | 25.4938 | 20.67 |
| 16 | 26.2430 | 36.19 |
| 17 | 28.0436 | 9.18 |
| 18 | 28.7361 | 21.80 |
| 19 | 30.1083 | 15.99 |
| 20 | 31.0739 | 7.82 |

In one embodiment of the present disclosure, the crystal form III exhibits an XRPD diffractogram according to Fig 1C when measured using Cu K_{α} radiation.

### Crystal form III: Melting points (endothermic events)

In addition to XRPD diffractograms, the present polymorphs are also defined by their melting points. The melting points can be determined as an endothermic event observed by differential scanning calorimetry (DSC) which is not associated with a mass loss. The melting point is defined by either the onset temperature or the peak temperature of the endothermic event, or both.

In one embodiment of the present disclosure, the crystal form III exhibits in differential scanning calorimetry (DSC) an onset temperature of from 220 to 228 °C using a heating rate of 10 °C per minute, such as from 221 to 226 °C, such as from 222 to 224 °C, for example 223 °C. In one embodiment of the present disclosure, the crystal form III exhibits in differential scanning calorimetry (DSC) an onset temperature of 223 °C using a heating rate of 10 °C per minute.

In one embodiment of the present disclosure, the crystal form III exhibits in differential scanning calorimetry (DSC) a peak temperature of from 225 to 235 °C using a heating rate of 10 °C per minute, such as from 226 to 234 °C, such as from 226 to 234 °C, such as from 227 to 233 °C, such as from 228 to 232 °C, such as from 229 to 231 °C, for example 230 °C.

In one embodiment of the present disclosure, the crystal form III exhibits in differential scanning calorimetry (DSC) a peak temperature of 230 °C using a heating rate of 10 °C per minute.

### Crystal form IV

A non-solvate crystal form of SCO-101 (Form IV) can be obtained e.g. by storage of crystal form III at 40°C/75% relative humidity (RH) in an open vial for 3 days as demonstrated by Example 5. The non-solvate crystal form of SCO-101 (Form IV) can in turn be transformed to crystal form I as demonstrated by Example 7.

Thus in one embodiment of the present disclosure, a crystal form IV of SCO-101 is provided: 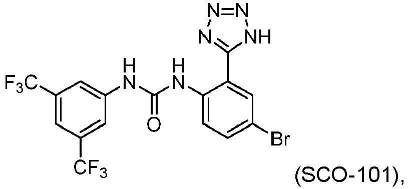 exhibiting at least peak maxima at 2 Theta angles: 22.6±0.2, 23.4±0.2, and 23.7±0.2 in an X-ray powder diffraction (XRPD) diffractogram when measured using Cu K_{α} radiation.

In one embodiment of the present disclosure, the crystal form IV exhibits at least peak maxima at 2 Theta angles: 21.6±0.2, 22.6±0.2, 23.4±0.2, 23.7±0.2, and 24.1±0.2 in an X-ray powder diffraction (XRPD) diffractogram when measured using Cu K_{α} radiation.

In one embodiment of the present disclosure, the crystal form IV further exhibits one or more peak maxima at 2 Theta angles selected from the group consisting of: 21.6±0.2, 24.1±0.2, and 27.2±0.2 in an X-ray powder diffraction (XRPD) diffractogram when measured using Cu K_{α} radiation.

In one embodiment of the present disclosure, the crystal form IV exhibits at least peak maxima at 2 Theta angles: 21.6±0.2, 22.6±0.2, 23.4±0.2, 23.7±0.2, 24.1±0.2, and 27.2±0.2 in an X-ray powder diffraction (XRPD) diffractogram when measured using Cu K_{α} radiation.

In one embodiment of the present disclosure, the crystal form IV exhibits at least peak maxima at 2 Theta angles: 21.6±0.2, 20.1±0.2, 22.6±0.2, 23.4±0.2, 23.7±0.2, 24.1±0.2, and 27.2±0.2 in an X-ray powder diffraction (XRPD) diffractogram when measured using Cu K_{α} radiation.

In one embodiment of the present disclosure, the crystal form IV exhibits at least peak maxima at 2 Theta angles: 21.6±0.2, 20.1±0.2, 22.6±0.2, 23.4±0.2, 23.7±0.2, 24.1±0.2, 25.6±0.2, and 27.2±0.2 in an X-ray powder diffraction (XRPD) diffractogram when measured using Cu K_{α} radiation.

In one embodiment of the present disclosure, the crystal form IV exhibits at least peak maxima at 2 Theta angles: 13.5±0.2, 21.6±0.2, 20.1±0.2, 22.6±0.2, 23.4±0.2, 23.7±0.2, 24.1±0.2, 25.6±0.2, and 27.2±0.2 in an X-ray powder diffraction (XRPD) diffractogram when measured using Cu K_{α} radiation.

In one embodiment of the present disclosure, the crystal form IV exhibits at least peak maxima at 2 Theta angles: 21.6±0.2, 22.6±0.2, 23.4±0.2, 23.7±0.2, and 24.1±0.2 in an X-ray powder diffraction (XRPD) diffractogram when measured using Cu K_{α} radiation.

In one embodiment of the present disclosure, the crystal form IV exhibits at least peak maxima at 2 Theta angles: 13.5±0.2, 20.1±0.2, 21.6±0.2, 22.6±0.2, 23.4±0.2, 23.7±0.2, 24.1±0.2, 25.6±0.2, 27.4±0.2, and 30.2±0.2 in an X-ray powder diffraction (XRPD) diffractogram when measured using Cu K_{α} radiation.

In one embodiment of the present disclosure, the crystal form IV exhibits at least peak maxima at 2 Theta angles according to table IV in an X-ray powder diffraction (XRPD) diffractogram when measured using Cu K_{α} radiation.

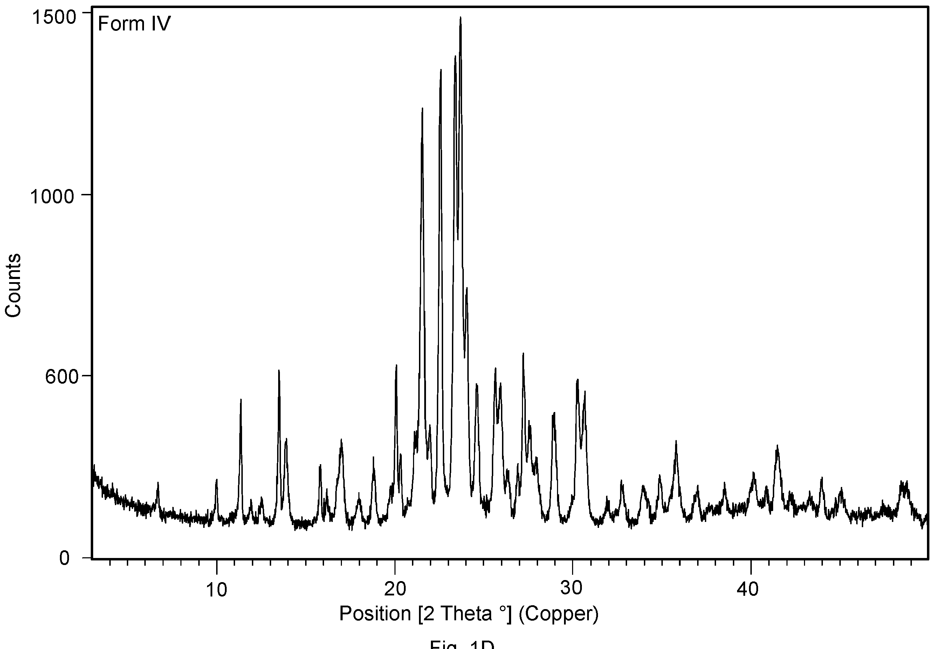

**Table IV: The 2-theta values and intensity for the 20 most intense peaks of crystal form IV, c.f. Fig. 1D.**

| **No.** | **Pos. [°2θ]** | **Rel. Int. [%]** |
|---|---|---|
| 1 | 11.3556 | 23.17 |
| 2 | 13.5131 | 29.44 |
| 3 | 13.8704 | 16.16 |
| 4 | 20.0964 | 31.38 |
| 5 | 20.3234 | 13.32 |
| 6 | 21.1111 | 16.87 |
| 7 | 21.5645 | 82.82 |
| 8 | 21.9786 | 18.10 |
| 9 | 22.5674 | 88.43 |
| 10 | 23.4016 | 93.09 |
| 11 | 23.7047 | 100.00 |
| 12 | 24.0659 | 43.32 |
| 13 | 24.6181 | 27.32 |
| 14 | 25.6391 | 30.09 |
| 15 | 25.9604 | 27.41 |
| 16 | 27.2077 | 33.26 |
| 17 | 27.5738 | 17.59 |
| 18 | 28.9019 | 20.22 |
| 19 | 30.2397 | 27.65 |
| 20 | 30.6414 | 24.05 |

In one embodiment of the present disclosure, the crystal form IV exhibits an XRPD diffractogram according to Fig 1D when measured using Cu K_{α} radiation.

### Crystal form IV: Melting points (endothermic events)

In addition to XRPD diffractograms, the present polymorphs are also defined by their melting points. The melting points can be determined as an endothermic event observed by differential scanning calorimetry (DSC) which is not associated with a mass loss. The melting point is defined by either the onset temperature or the peak temperature of the endothermic event, or both.

In one embodiment of the present disclosure, the crystal form IV exhibits in differential scanning calorimetry (DSC) an onset temperature of from 222 to 230 °C using a heating rate of 10 °C per minute, such as from 223 to 228 °C, such as from 224 to 226 °C, for example 225 °C. In one embodiment of the present disclosure, the crystal form IV exhibits in differential scanning calorimetry (DSC) an onset temperature of 225 °C using a heating rate of 10 °C per minute.

In one embodiment of the present disclosure, the crystal form IV exhibits in differential scanning calorimetry (DSC) a peak temperature of from 226 to 236 °C using a heating rate of 10 °C per minute, such as from 227 to 235 °C, such as from 228 to 234 °C, such as from 229 to 233 °C, such as from 230 to 232 °C, for example 231 °C. In one embodiment of the present disclosure, the crystal form IV exhibits in differential scanning calorimetry (DSC) a peak temperature of 231 °C using a heating rate of 10 °C per minute.

### Crystal form V

A crystalline isopropanol solvate of SCO-101 was identified as crystal form V. Crystal form V can be converted into crystal form I as demonstrated in Example 7.

In one embodiment of the present disclosure, a crystal form V of SCO-101 isopropanol solvate is provided: 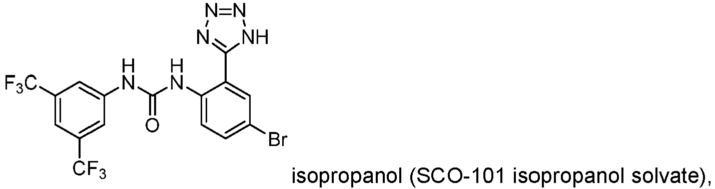 exhibiting at least peak maxima at 2 Theta angles: 9.4±0.2, 21.1±0.2, and 22.2±0.2 in an X-ray powder diffraction (XRPD) diffractogram when measured using Cu K_{α} radiation.

In one embodiment of the present disclosure, the crystal form V further exhibits one or more peak maxima at 2 Theta angles selected from the group consisting of: 8.2±0.2, 10.5±0.2, and 24.2±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 21.1±0.2, and 22.2±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 10.5±0.2, 21.1±0.2, and 22.2±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 10.5±0.2, 21.1±0.2, 22.2±0.2, and 24.2±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 10.5±0.2, 21.1±0.2, 22.2±0.2, 24.2±0.2, and 28.5±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 10.5±0.2, 21.1±0.2, 22.2±0.2, 24.2±0.2, 25.5±0.2, and 28.5±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 10.5±0.2, 21.1±0.2, 22.2±0.2, 24.2±0.2, 24.6±0.2, 25.5±0.2, and 28.5±0.2 in an X-ray powder diffraction (XRPD) diffractogram when measured using Cu K_{α} radiation.

In one embodiment of the present disclosure, the crystal form V exhibits at least peak maxima at 2 Theta angles: 8.2±0.2, 9.4±0.2, 10.5±0.2, 21.1±0.2, 22.2±0.2, 23.7±0.2, 24.2±0.2, 24.6±0.2, 25.5±0.2, and 28.5±0.2 in an X-ray powder diffraction (XRPD) diffractogram when measured using Cu K_{α} radiation.

In one embodiment of the present disclosure, the crystal form V exhibits at least peak maxima at 2 Theta angles according to table V in an X-ray powder diffraction (XRPD) diffractogram when measured using Cu K_{α} radiation.

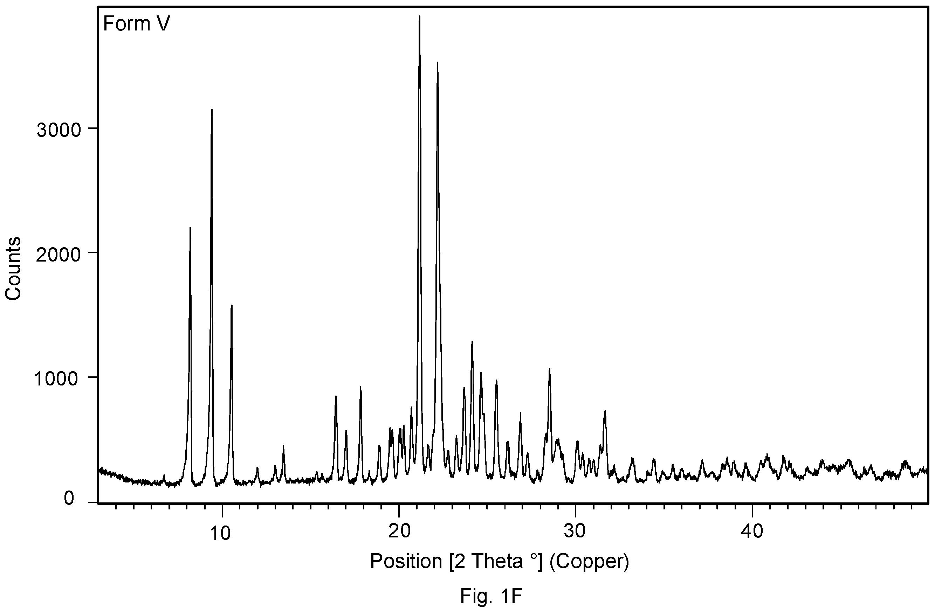

**Table V: The 2-theta values and intensity for the 20 most intense peaks of crystal form V, c.f. Fig. 1F.**

| No. | Pos. [°2θ] | Rel. Int. [%] |
|---|---|---|
| 1 | 8.2129 | 52.37 |
| 2 | 9.4091 | 79.34 |
| 3 | 10.5323 | 38.59 |
| 4 | 16.4408 | 18.93 |
| 5 | 17.8586 | 19.69 |
| 6 | 19.4907 | 11.32 |
| 7 | 20.0585 | 11.76 |
| 8 | 20.2728 | 12.23 |
| 9 | 20.7038 | 16.41 |
| 10 | 21.1492 | 100.00 |
| 11 | 22.1772 | 90.59 |
| 12 | 23.6773 | 20.13 |
| 13 | 24.1579 | 30.45 |
| 14 | 24.6082 | 20.73 |
| 15 | 24.8297 | 13.37 |
| 16 | 25.4742 | 18.83 |
| 17 | 25.5306 | 22.01 |
| 18 | 26.8722 | 13.93 |
| 19 | 28.5280 | 24.11 |
| 20 | 31.6350 | 14.51 |

In one embodiment of the present disclosure, the crystal form V exhibits an XRPD diffractogram according to Fig 1F when measured using Cu K_{α} radiation.

### Crystal form V: Melting points (endothermic events)

In addition to XRPD diffractograms, the present polymorphs are also defined by their melting points. The melting points can be determined as an endothermic event observed by differential scanning calorimetry (DSC) which is not associated with a mass loss. The melting point is defined by either the onset temperature or the peak temperature of the endothermic event, or both.

In one embodiment of the present disclosure, the crystal form V exhibits in differential scanning calorimetry (DSC) an onset temperature of from 216 to 224 °C using a heating rate of 10 °C per minute, such as from 217 to 223 °C, such as from 218 to 222 °C, such as from 219 to 221 °C, for example 220 °C. In one embodiment of the present disclosure, the crystal form V exhibits in differential scanning calorimetry (DSC) an onset temperature of 220 °C using a heating rate of 10 °C per minute.

In one embodiment of the present disclosure, the crystal form V exhibits in differential scanning calorimetry (DSC) a peak temperature of from 224 to 234 °C using a heating rate of 10 °C per minute, such as from 225 to 233 °C, such as from 226 to 232 °C, such as from 227 to 231 °C, such as from 228 to 230 °C, for example 229 °C. In one embodiment of the present disclosure, the crystal form V exhibits in differential scanning calorimetry (DSC) a peak temperature of 229 °C using a heating rate of 10 °C per minute.

### Amorphous SCO-101

Amorphous SCO-101 can be prepared as demonstrated in Example 2.

In one embodiment of the present disclosure, an amorphous form of SCO-101 is provided: 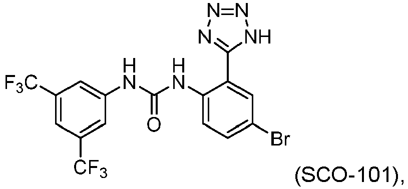 exhibiting no peak maxima at 2 Theta angles between 0 and 40 in an X-ray powder diffraction (XRPD) diffractogram when measured using Cu K_{α} radiation.

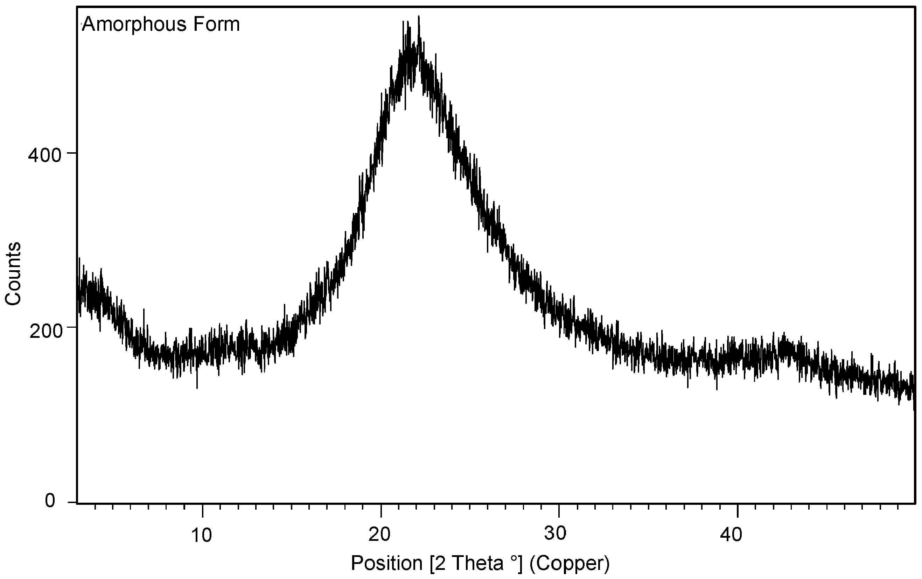

While the XRPD diffractograms of amorphous materials, such as the amorphous form of SCO-101 are not strictly horizontal, the person of skill in the art will know that the convex baseline as in Fig. 1E does not represent a peak maximum.

In one embodiment of the present disclosure, the amorphous form exhibits an XRPD diffractogram according to Fig 1E when measured using Cu K_{α} radiation.

In one embodiment of the present disclosure, the amorphous form exhibits in differential scanning calorimetry (DSC) an onset temperature of from 211 to 219 °C using a heating rate of 10 °C per minute, such as from 212 to 218 °C, such as from 213 to 217 °C, such as from 214 to 216 °C, for example 215 °C. In one embodiment of the present disclosure, the amorphous form exhibits in differential scanning calorimetry (DSC) an onset temperature of 215 °C using a heating rate of 10 °C per minute.

In one embodiment of the present disclosure, the amorphous form exhibits in differential scanning calorimetry (DSC) a peak temperature of from 218 to 228 °C using a heating rate of 10 °C per minute, such as from 219 to 227 °C, such as from 220 to 226 °C, such as from 221 to 225 °C, such as from 222 to 224 °C, for example 223 °C.

In one embodiment of the present disclosure, the amorphous form exhibits in differential scanning calorimetry (DSC) a peak temperature of 223 °C using a heating rate of 10 °C per minute.

### Metastable forms of SCO-101

In one embodiment of the present disclosure, a metastable form of SCO-101 is provided: 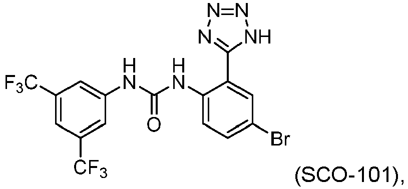 which transforms into crystal form I under storage or by the process as defined herein for the preparation of crystal form I; wherein the metastable form is not crystal form II of SCO-101 exhibiting at least peak maxima at 2 Theta angles: 18.8±0.2, 23.2±0.2, and 20.5±0.2 in an X-ray powder diffraction (XRPD) diffractogram when measured using Cu K_{α} radiation. The metastable forms forming part of the present invention are those recited specifically in the appended claims, namely crystal forms III, IV, V and the amorphous form.

In one embodiment of the present disclosure, under storage refers to storage at room temperature, such as 20°C - 25°C. In one embodiment of the present disclosure, under storage refers to storage at from 35 to 50°C. In one embodiment of the present disclosure, under storage refers to storage at from 2°C to 8°C.

In one embodiment of the present disclosure, under storage refers to storage for one month following the manufacture of the metastable form. In one embodiment of the present disclosure, under storage refers to storage for one month or more, such as two months or more, such as three months or more, such as four months or more, such as five months or more, such as six months or more following the manufacture of the metastable form.

### Preparation of crystal form I

### Crystal form I: Preparation

SCO-101 can be prepared using the method described in Example 1. The SCO-101 product obtained using the method of Example 1 is crystal form II, which can be converted to crystal form I, e.g. using the method described in Example 4 or the method described in Example 7. In one embodiment of the present disclosure, SCO-101 used in the preparation of crystal form I is dried, optionally *in vacuo,* prior to step a).

In one embodiment of the present disclosure, a process is provided for preparing a crystal form I of SCO-101 as defined herein, the process comprising the consecutive steps of:
a) dissolving SCO-101 in one or more polar aprotic solvents at a first predefined temperature;
b) adding one or more polar protic solvents to the one or more polar aprotic solvents over the course of a first predefined time period to provide the crystal form I of SCO-101; and
c) isolating the crystal form I of SCO-101.

In one embodiment of the present disclosure, a process is provided for preparing a crystal form I of SCO-101 as defined herein, the process comprising the consecutive steps of:
a) dissolving SCO-101 in one or more polar protic solvents at a first predefined temperature;
b) adding one or more polar aprotic solvents to the one or more polar protic solvents over the course of a first predefined time period to provide the crystal form I of SCO-101; and
c) isolating the crystal form I of SCO-101.

In one embodiment of the present disclosure, a process is provided for preparing a crystal form I of SCO-101 as defined herein, the process comprising the consecutive steps of:
a) dissolving SCO-101 in one or more polar aprotic solvents at a first predefined temperature;
b) adding one or more apolar solvents to the one or more polar aprotic solvents over the course of a first predefined time period to provide the crystal form I of SCO-101; and
c) isolating the crystal form I of SCO-101.

In one embodiment of the present disclosure, the process further comprises a prior crystallization step which is prior to step a), wherein the prior crystallization step comprises:
i) mixing a composition comprising SCO-101 and one or more impurities with 2-propanol to provide a mixture;
ii) heating the mixture to or at a second predefined temperature above the first predefined temperature;
iii) adding water to the mixture over the course of a second predefined time period;
iv) cooling the mixture to a third predefined temperature below the second predefined temperature to provide SCO-101 as a solid, optionally further isolating the solid by filtration.

### Solvent

In one embodiment of the present disclosure, the one or more polar aprotic solvents are selected from the group consisting of: acetone, acetonitrile, dichloromethane, dimethylformamide, dimethylpropyleneurea, dimethylsulfoxide, ethyl acetate, 2-MeTHF, and tetrahydrofuran. In one embodiment of the present disclosure, the polar aprotic solvent is acetone.

In one embodiment of the present disclosure, the one or more polar protic solvents are selected from the group consisting of: water, methanol, ethanol, isopropanol, and acetic acid. In one embodiment of the present disclosure, the polar protic solvent is water.

In one embodiment of the present disclosure, the one or more apolar solvents are selected from the group consisting of: pentane, heptane, cyclohexane, and methyl cyclohexane.

### Temperature

In one embodiment of the present disclosure, the first predefined temperature is from 0 to 20 °C, such as 10 ± 5 °C.

In one embodiment of the present disclosure, the second predefined temperature is from 31 to 80 °C, such as from 31 to 35 °C, such as from 35 to 40 °C, such as from 40 to 45 °C, such as from 45 to 50 °C, such as from 50 to 55 °C, such as from 55 to 60 °C, such as from 60 to 65 °C, such as from 65 to 70 °C, such as from 70 to 75 °C, such as from 75 to 80 °C, for example 50 °C.

In one embodiment of the present disclosure, the third predefined temperature is from 10 to 30 °C, such as from 10 to 12 °C, such as from 12 to 14 °C, such as from 14 to 16 °C, such as from 16 to 18 °C, such as from 18 to 20 °C, such as from 20 to 22 °C, such as from 22 to 24 °C, such as from 24 to 26 °C, such as from 26 to 28 °C, such as from 28 to 30 °C, for example 20 °C.

### Time

In one embodiment of the present disclosure, the first predefined time period is from 10 to 360 minutes, such as from 70 to 90 minutes.

In one embodiment of the present disclosure, wherein the second predefined time period is from 1 to 120 minutes, such as from 1 to 10 minutes, such as from 10 to 20 minutes, such as from 20 to 30 minutes, such as from 30 to 40 minutes, such as from 40 to 50 minutes, such as from 50 to 60 minutes, such as from 60 to 70 minutes, such as from 70 to 80 minutes, such as from 80 to 90 minute, such as from 90 to 100 minutes, such as from 100 to 110 minutes, such as from 110 to 120 minutes. In one embodiment of the present disclosure, the second predefined time period is 30 minutes.

### Impurities

In one embodiment of the present disclosure, the one or more impurities are selected from the group consisting of: 4-bromo-2-(1H-1,2,3,4-tetraazol-5-yl)aniline, 3,5-bis(trifluoromethyl)-phenyl isocyanate, and toluene. One or more or all of these impurities can effectively be removed by the process disclosed herein e.g. as demonstrated by Example 4.

### Seeding

In one embodiment of the present disclosure, the process disclosed herein comprising adding one or more seed crystals of the crystal form I of SCO-101. The one or more seed crystals can be added prior to, during or subsequent to any of the steps disclosed herein in the preparation of crystal form I of SCO-101. In on embodiment, the one or more seed crystals are added prior to step a) of the process disclosed herein.

In one embodiment of the present disclosure, the process is provided where no seed crystals are added.

### Preparation of crystal form I from metastable forms

The slurry experiments of Example 7 demonstrate that the crystalline, non-solvated crystal form I of SCO-101 is the thermodynamically stable polymorph. In addition, these methods/processes can be used to transform metastable forms into crystal form I.

In one embodiment of the present disclosure, a process is provided for preparing the crystal form I of SCO-101 as defined herein from a metastable form, comprising:
a) providing a metastable form which is a crystal form or an amorphous form of SCO-101;
b) mixing the metastable form with the crystal form I of SCO-101 as defined herein in a solvent mixture of: i) one or more polar aprotic solvents and ii) one or more polar protic solvents or one or more apolar solvents;
c) agitating the solvent mixture at a sixth predefined temperature for at least 1 hour thereby providing the crystal form I of SCO-101.

In one embodiment of the present disclosure, the solvent mixture in step c) is agitated for 6 hours or more, such as 12 hours or more, such as 1 day or more, such as 2 days or more, such as 3 days or more, such as 4 days or more, such as 5 days or more, such as 6 days or more, such as 7 days or more.

In one embodiment of the present disclosure, the crystal form I of SCO-101 obtained in step c) is separated from the solvent mixture, optionally by filtration.

### Metastable forms

In one embodiment of the present disclosure, the metastable form of SCO-101 is selected from the group consisting of:
i) a crystal form II of SCO-101: 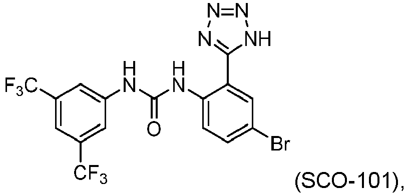 exhibiting at least peak maxima at 2 Theta angles: 18.8±0.2, 23.2±0.2, and 20.5±0.2 in an X-ray powder diffraction (XRPD) diffractogram when measured using Cu K_{α} radiation;
ii) a crystal form III of SCO-101 as defined herein;
iii) a crystal form IV of SCO-101 as defined herein; and
iv) a crystal form V of SCO-101 as defined herein.

### Temperature

In one embodiment of the present disclosure, the sixth predefined temperature is from 30 to 60 °C, such as from 30 to 32 °C, such as from 32 to 34 °C, such as from 34 to 36 °C, such as from 36 to 38 °C, such as from 38 to 40 °C, such as from 40 to 42 °C, such as from 42 to 44 °C, such as from 44 to 46 °C, such as from 46 to 48 °C, such as from 48 to 50 °C, such as from 50 to 52 °C, such as from 52 to 54 °C, such as from 54 to 56 °C, such as from 56 to 58 °C, such as from 58 to 60 °C, for example 40 °C.

In one embodiment of the present disclosure, the sixth predefined temperature is from 30 to 60 °C, such as from 31 to 58 °C, such as from 32 to 56 °C, such as from 33 to 54 °C, such as from 34 to 52 °C, such as from 35 to 50 °C, such as from 36 to 48 °C, such as from 37 to 46 °C, such as from 38 to 44 °C, such as from 39 to 42 °C, for example 40 °C.

In one embodiment of the present disclosure, the solvent mixture in step c) is agitated for 6 hours or more, such as 12 hours or more, such as 1 day or more, such as 2 days or more, such as 3 days or more, such as 4 days or more, such as 5 days or more, such as 6 days or more, such as 7 days or more.

### Solvent

In one embodiment of the present disclosure, the one or more polar aprotic solvents are selected from the group consisting of: acetone, acetonitrile, dichloromethane, dimethylformamide, dimethylpropyleneurea, dimethylsulfoxide, ethyl acetate, 2-MeTHF, and tetrahydrofuran.

In one embodiment of the present disclosure, wherein the one or more polar protic solvents are selected from the group consisting of: water, methanol, ethanol, isopropanol, and acetic acid.

In one embodiment of the present disclosure, the one or more apolar solvents are selected from the group consisting of: heptane, hexane, pentane, cyclohexane, toluene, and diethyl ether.

In one embodiment of the present disclosure, the polar aprotic solvent is acetone and the polar protic solvent is water. In one embodiment of the present disclosure, the polar aprotic solvent is acetone and the apolar solvent is heptane.

In one embodiment of the present disclosure, a crystal form I of SCO-101 is provided: 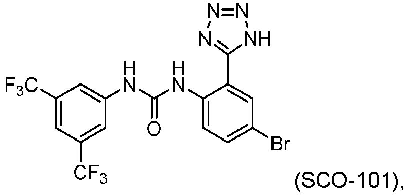 obtainable by the process as defined herein for preparing the crystal form I of SCO-101.

### Crystal form III: Preparation

In one embodiment of the present disclosure, a process is provided for preparing a crystal form III of SCO-101 as defined herein, the process comprising the consecutive steps of:
a) mixing SCO-101 with one or more polar protic solvents, such as methanol to provide a mixture;
b) performing one or more temperature cycles, wherein the temperature is cycled between a fourth predefined temperature and a fifth predefined temperature, wherein the fourth predefined temperature is higher than the fifth predefined temperature;
c) isolating a crystal form III of SCO-101 as defined herein from the mixture.

In one embodiment of the present disclosure, SCO-101 in step a) is an amorphous form of SCO-101 as defined herein.

### Temperature cycles

In one embodiment of the present disclosure, the one or more temperature cycles is from 2 to 6 cycles, such as from 2 to 3 cycles, such as from 3 to 4 cycles, such as from 4 to 5 cycles, such as from 5 to 6 cycles.

### Temperature

In one embodiment of the present disclosure, the fourth predefined temperature is from 31 to 60 °C, such as from 31 to 35 °C, such as from 35 to 40 °C, such as from 40 to 45 °C, such as from 45 to 50 °C, such as from 50 to 55 °C, such as from 55 to 60 °C.

In one embodiment of the present disclosure, the fifth predefined temperature is from 15 to 30 °C, such as from 15 to 20 °C, such as from 20 to 25 °C, such as from 25 to 30 °C.

### Drying

In one embodiment of the present disclosure, SCO-101 is dried, optionally in vacuo, prior to step c). In one embodiment of the present disclosure, SCO-101 is dried, optionally in vacuo, after step c).

In one embodiment of the present disclosure, SCO-101 is dried, optionally in vacuo, at from 30 to 60 °C for at least 6 hours prior to step c). In one embodiment of the present disclosure, SCO-101 is dried, optionally in vacuo, at from 30 to 60 °C for at least 6 hours after step c).

In one embodiment of the present disclosure, SCO-101 is dried, optionally in vacuo, at from 30 to 60 °C for from 6 hours to 72 hours prior to step c). In one embodiment of the present disclosure, SCO-101 is dried, optionally in vacuo, at from 30 to 60 °C for from 6 hours to 72 hours after step c).

### Crystal form IV: Preparation

In one embodiment of the present disclosure, a process is provided for preparing a crystal form IV of SCO-101 as defined herein, the process comprising the consecutive steps of:
a) providing a crystal form III of SCO-101 as defined herein;
b) storing the crystal form III of SCO-101 at from 30 °C to 60 °C for at least 24 hours thereby preparing the crystal form IV of SCO-101.

In one embodiment of the present disclosure, the crystal form III of SCO-101 is stored for 1 day or more, such as 2 days or more, such as 3 days or more, such as 4 days or more, such as 5 days or more, such as 6 days or more, such as 7 days or more.

### Crystal form V: Preparation

Crystal form V can be provided as an intermediary crystal form towards crystal form I. In particular, the first part of the method as described in Example 4 prior to solvation of SCO-101 in acetone can be used to provide crystal form V. Crystal form V can be further converted to the improved form, crystal form I using the methods described herein and in particular as exemplified by Example 4. Crystal form V can also be converted to crystal form I as exemplified by Example 7.

In one embodiment of the present disclosure, a process for preparing a crystal form V of SCO-101 as defined in herein is provided, the process comprising the consecutive steps of:
a) mixing a composition comprising SCO-101 with isopropanol to provide a mixture;
b) heating the mixture to or at a seventh predefined temperature;
c) adding water to the mixture over a third predefined time period;
d) adjusting the temperature of the mixture to an eighth predefined temperature over a fourth predefined time period to provide a solid composition;
e) isolating the solid composition, and optionally drying the solid composition, to provide the crystal form V of SCO-101.

### Temperature

In one embodiment of the present disclosure, the seventh predefined temperature is from 30 to 82 °C, such as from 31 to 80 °C, such as from 32 to 78 °C, such as from 33 to 76 °C, such as from 34 to 74 °C, such as from 35 to 72 °C, such as from 36 to 70 °C, such as from 38 to 68 °C, such as from 39 to 66 °C, such as from 40 to 64 °C, such as from 41 to 62 °C, such as from 42 to 60 °C, such as from 43 to 58 °C, such as from 44 to 56 °C, such as from 45 to 55 °C, such as from 46 to 54 °C, such as from 47 to 52 °C, such as from 48 to 52 °C, such as from 49 to 51 °C, for example 50 °C.

In one embodiment of the present disclosure, the eighth predefined temperature is from 2 to 29 °C, such as from 4 to 28 °C, such as from 6 to 27 °C, such as from 8 to 26 °C, such as from 10 to 25 °C, such as from 12 to 24 °c, such as from 14 to 24 °C, such as from 16 to 24 °C, such as from 17 to 23 °C, such as from 18 to 22 °C, such as from 19 to 21 °C, for example 20 °C.

### Time

In one embodiment of the present disclosure, the third predefined time period is within 1 hour, such as within 50 minutes, such as within 40 minutes, such as within 30 minutes.

In one embodiment of the present disclosure, the third predefined time period is from 1 minute to 60 minutes, such as from 5 minutes to 55 minutes, such as from 10 minutes to 50 minutes, such as from 15 minutes to 45 minutes, such as from 20 minutes to 40 minutes, such as from 25 minutes to 35 minutes, for example 30 minutes.

In one embodiment of the present disclosure, the fourth predefined time period is 1 minute or more, such as 10 minutes or more, such as 20 minutes or more, such as 30 minutes or more such as 40 minutes or more, such as 50 minutes or more, such as 60 minutes or more.

In one embodiment of the present disclosure, the fourth predefined time period is from 1 minute to 12 hours, such as from 5 minutes to 10 hours, such as from 10 minutes to 8 hours, such as from 20 minutes to 6 hours, such as from 20 minutes to 4 hours, such as from 30 minutes to 2 hours, for example 1 hour.

### Amorphous SCO-101: Preparation

In one embodiment of the present disclosure, a process for preparing an amorphous form of SCO-101 as defined herein is provided, the process comprising the consecutive steps of:
a) mixing a composition comprising SCO-101 with one or more polar aprotic solvents to obtain a clear solution which has no visible solid material indicating full dissolution of the composition;
b) evaporating the one or more polar aprotic solvents at a ninth predefined temperature, optionally *in vacuo,* to provide a solid composition;
c) optionally further drying the solid composition, providing the amorphous form of SCO-101.

### Solvent

In one embodiment of the present disclosure, the one or more polar aprotic solvents are selected from the group consisting of: acetone, acetonitrile, dichloromethane, dimethylformamide, dimethylpropyleneurea, dimethylsulfoxide, ethyl acetate, 2-MeTHF, and tetrahydrofuran. In one embodiment of the present disclosure, the polar aprotic solvent is acetone.

### Temperature

In one embodiment of the present disclosure, the ninth predefined temperature is at the boiling point of the one or more polar aprotic solvents.

In one embodiment of the present disclosure, the ninth predefined temperature is at from 30 to 60 °C, such as from 31 to 58 °C, such as from 32 to 56 °C, such as from 33 to 54 °C, such as from 34 to 52 °C, such as from 35 to 50 °C, such as from 36 to 48 °C, such as from 37 to 46 °C, such as from 38 to 44 °C, such as from 39 to 42 °C, for example 40 °C.

### Pharmaceutical compositions

One embodiment of the present disclosure provides for a pharmaceutical composition as disclosed herein, wherein the pharmaceutical composition is formulated for oral administration. Such composition may be in the form of a tablet or a capsule.

In one embodiment of the present disclosure, a pharmaceutical composition is provided comprising any one of the crystal forms or amorphous forms of SCO-101 as defined herein; and one or more pharmaceutically acceptable adjuvants, excipients, carriers, buffers and/or diluents.

In one embodiment of the present disclosure, a pharmaceutical composition is provided comprising any one of the crystal forms or amorphous forms of SCO-101 as defined herein with the proviso that the crystal form is not crystal form II of SCO-101; and one or more pharmaceutically acceptable adjuvants, excipients, carriers, buffers and/or diluents.

In one embodiment of the present disclosure, a pharmaceutical composition is provided comprising any one of crystal forms I, III, IV, V or amorphous forms of SCO-101 as defined herein; and one or more pharmaceutically acceptable adjuvants, excipients, carriers, buffers and/or diluents.

In one embodiment of the present disclosure, a pharmaceutical composition is provided comprising a crystal form I of SCO-101 as defined herein; and one or more pharmaceutically acceptable adjuvants, excipients, carriers, buffers and/or diluents.

### Crystal form I in cancer treatment

In one embodiment of the present disclosure, a pharmaceutical composition as defined herein is provided for use in treating a patient having cancer in combination with one or more anti-cancer agents. A therapeutic effect can be obtained by administering the pharmaceutical composition comprising any crystal form or amorphous form of SCO-101 in combination with one or more of the anti-cancer agents disclosed herein.

In one embodiment of the present disclosure, a pharmaceutical composition as defined herein comprising a crystal form I of SCO-101 as defined herein is provided for use in treating a patient having cancer in combination with one or more anti-cancer agents.

In one embodiment of the present disclosure, the crystal form I of SCO-101 as defined herein, or a pharmaceutical composition as defined herein is provided for treatment of cancer as defined herein, wherein the crystal form I of SCO-101 as defined herein, or a pharmaceutical composition as defined herein is administered to the patient daily.

In one embodiment, the present disclosure provides the crystal form I of SCO-101 as defined herein for use in the treatment of cancer, wherein the crystal form I of SCO-101 is administered in combination with one or more anti-cancer agents.

In one embodiment of the present disclosure, the one or more anticancer agents may be administered simultaneously, sequentially, or separately from SCO-101.

In one embodiment of the present disclosure, the one or more anti-cancer agents are selected from the group consisting of topoisomerase inhibitors, anti-hormone agents, alkylating agents, mitotic inhibitors, antimetabolites, anti-tumor antibiotics, corticosteroids, targeted anti-cancer therapy, differentiating agents and immunotherapy.

### Topoisomerase inhibitors

In one embodiment of the present disclosure, the anti-cancer agent is a topoisomerase I inhibitor or topoisomerase II inhibitor.

In one embodiment of the present disclosure, the anti-cancer agent is a topoisomerase I inhibitor selected from the group consisting of: irinotecan, its active metabolite SN-38, and topotecan.

### Anti-hormone agents

In one embodiment of the present disclosure, anti-cancer agent is an anti-hormone agent which is:
a. an anti-estrogen selected from the group consisting of: fulvestrant, tamoxifen, toremifene, and clomifene, or
b. an anti-progestogen selected from the group consisting of: mifepristone, ulipristal acetate, aglepristone, lilopristone and onapristone.

In one embodiment of the present disclosure, the anti-estrogen is fulvestrant or tamoxifen.

In one embodiment of the present disclosure, the anti-progestogen is onapristone.

### Alkylating agents

In one embodiment of the present disclosure, the anti-cancer agent is an alkylating agent which is:
a. a nitrogen mustard selected from the group consisting of: mechlorethamine, chlorambucil, cyclophosphamide, ifosfamide, and melphalan,
b. a nitrosourea selected from the group consisting of: streptozocin, carmustine, and lomustine,
c. an alkyl sulfonate selected from the group consisting of: busulfan,
d. a triazine selected from the group consisting of: dacarbazine (DTIC) and temozolomide, or
e. an ethylenimine selected from the group consisting of: thiotepa and altretamine (hexamethylmelamine).

In one embodiment of the present disclosure, the alkylating agent is temozolomide.

### Antimetabolites

In one embodiment of the present disclosure, the anti-cancer agent is an antimetabolite selected from the group consisting of: 5-fluorouracil, 6-mercaptopurine, Capecitabine, Cytarabine, Floxuridine, Fludarabine, Gemcitabine, Hydroxyurea, Methotrexate, and Pemetrexed.

In one embodiment of the present disclosure, the antimetabolite is 5-fluorouracil or gemcitabine.

### Mitotic inhibitors

In one embodiment of the present disclosure, the anti-cancer agent is a mitotic inhibitor which is:
a. a taxane selected from the group consisting of: paclitaxel and docetaxel;
b. ixabepilone;
c. a vinca alkaloid selected from the group consisting of: vinblastine, vincristine, and vinorelbine; or
d. estramustine.

In one embodiment of the present disclosure, the mitotic inhibitor is paclitaxel or docetaxel.

### Further anti-cancer agents

In one embodiment of the present disclosure, the anti-cancer agent is administered in combination with one or more further anti-cancer agents.

In one embodiment of the present disclosure, the anti-cancer agent is administered in combination with a further anti-cancer agent which is 5-fluorouracil. In one embodiment of the present disclosure, the anti-cancer agent is administered in combination with 5-fluorouracil and folinic acid. In one embodiment of the present disclosure, the anti-cancer agent is irinotecan and administered in combination with 5-fluorouracil and folinic acid.

### Immunotherapy

In one embodiment of the present disclosure the anti-cancer agent is an immunotherapy agent. Immunotherapy drugs are given to people with cancer to help their immune systems recognize and attack cancer cells.

There are different types of immunotherapy. Active immunotherapies stimulate the body's own immune system to fight the disease. Passive immunotherapies do not rely on the body to attack the disease; they're immune system components (such as antibodies) created outside the body and given to fight the cancer.

Examples of active immunotherapies include:
· Monoclonal antibody therapy, such as rituximab (Rituxan^{®}) and alemtuzumab (Campath^{®})
· Non-specific immunotherapies and adjuvants (other substances or cells that boost the immune response), such as BCG, interleukin-2 (IL-2), and interferon-alfa
· Immunomodulating drugs, such as thalidomide and lenalidomide (Revlimid^{®})

In one embodiment of the present disclosure the anti-cancer agent is a PD-1 or PD-L1 inhibitor, such as an antibody capable of inhibiting PD-1 or PD-L1.

Cancer vaccines are a type of active specific immunotherapy.

### Cancers

In one embodiment of the present disclosure, the cancer is a solid tumour or a leukemia.

In one embodiment of the present disclosure, the cancer is a solid tumour, such as a solid tumour selected from sarcoma, carcinoma and lymphoma.

In one embodiment of the present disclosure, the cancer is selected from the group consisting of Colorectal cancer, Breast cancer, Lung cancer (non small cell Lung cancer and small cell Lung cancer), Glioblastomas, Head and Neck cancers, Malignant melanomas, Basal cell skin cancer, Squamous cell skin cancer, Liver cancer, Pancreatic cancer, Prostate cancer, Anal cancer, Cervix uteri cancer, Bladder cancer, Corpus uteri cancer, Ovarian cancer, Gall bladder cancer, Sarcomas, Leukemia's (myeloid and lymphatic), Lymphomas, Myelomatosis, and cholangiocarcinoma.

In one embodiment of the present disclosure, the cancer is metastatic cancer. In one embodiment of the present disclosure, the cancer is colorectal cancer, such as metastatic colorectal cancer. In one embodiment of the present disclosure, the cancer is pancreatic cancer, such as metastatic pancreatic cancer. In one embodiment of the present disclosure, the cancer is breast cancer, such as metastatic breast cancer.

In one embodiment of the present disclosure, the leukemia is acute myeloid leukemia (AML).

In one embodiment of the present disclosure, the cancer is a resistant cancer which is resistant to the anti-cancer agent when administered alone. In one embodiment of the present disclosure, resistance is *de novo* resistance or acquired resistance.

### Examples

### Example 1: Preparation of SCO-101 crude (Form II)

### Materials

The following materials were used. 4-Bromo-2-(1*H*-1,2,3,4-tetraazol-5-yl)aniline was manufactured in-house and 3,5-Bis(trifluoromethyl)-phenyl isocyanate was purchased from DONA FINE CHEMICALS S.J.

### Method

To a stirred solution of 4-Bromo-2-(1H-1,2,3,4-tetraazol-5-yl)aniline (3.2 kg) in toluene (62 L), 3,5-bis(trifluoromethyl)-phenyl isocyanate (3.5 kg) was added. The tubing used for addition of 3,5-bis(trifluoromethyl)-phenyl isocyanate was washed with toluene (3.8) L which was added to the reactor. The reaction mixture was heated to 55°C and stirred for 11 hrs. In Process Control (IPC) confirmed the conversion of the starting materials and the reaction mixture was cooled to 23°C over 2½ hours. The resulting suspension was filtered, the filter cake was washed with toluene (17 L), and dried at 45°C for 16 hrs. to provide a solid composition.

### Results

6.5 kg of SCO-101 was obtained as a crystalline material which was analysed by XRPD (Fig. 1B), DVS (Fig. 3B), and DSC-TGA (Fig. 2B). The crystalline form obtained is a metastable form, Form II.

### Conclusion

SCO-101 can be prepared in a metastable form, Form II using the method described in the present example. Form II is not the most stable crystal form of SCO-101 and takes up water to form a hydrate at increased relative humidity (RH), such as at from 70% RH and up. This makes form II of SCO-101 inferior from a clinical development point of view, compared to the thermostable form, Form I.

### Example 2: Preparation of amorphous SCO-101

### Materials

The following materials were used. SCO-101 Form I was manufactured in 3 L scale using the process described in Example 4. A batch of 268 g was obtained.

### Method

3 g of SCO-101 Form I was dissolved in 30 mL of acetone using sonication to aid dissolution. The clear solution was syringe filtered into a 50 mL round bottom flask and the solvent was removed by rotary evaporation over a water bath at 40 °C, and left on the rotary evaporator for a further hour to ensure the material was dried fully. A solid was obtained. The solid was decanted into a crystallisation dish, covered with tissue paper and dried in a vacuum oven at 40 °C under vacuum overnight (ca. 17 hours). The dried solid was analysed by XRPD which confirmed that amorphization was successful (c.f. Fig. 1E).

### Results

Amorphous SCO-101 can be prepared using the present example.

### Example 3: Polymorph screening of SCO-101

### Method A: solubility testing and evaporation

To approx. 10 mg amorphous SCO-101, aliquots of the listed solvents were added until dissolution was noted thereby obtaining a clear solution (continued in Method A2), or 1 mL had been added to form a slurry (continued in Method A1). The samples were manually agitated and heated to approx. 40°C in a block attached to a water batch in between each aliquot.

Method A1: The slurries were stored at room temperature overnight and then filtered centrifugally.

Method A2: Clear solutions were uncapped and allowed to evaporate at room temperature.

Any solids obtained from slurries (Method A1) or from evaporation (Method A2) were analysed by XRPD.

### Method B: solvent drop grinding

10 µl of the appropriate saturated solution (where available) or solvent was added to 20 mg of the amorphous SCO-101 in a 2 mL bead mill vial along with 2 steel bill balls.

The vials were bead milled using the following program:
- Speed: 6000 rpm
- Cycle: 40 × 90 s
- Pause: 10 s

### Method C: temperature cycling

Slurries of amorphous SCO-101 were prepared in vials.

All vials were capped, sealed with parafilm and temperature cycled between room temperature and 40°C over 4 hour cycles in an incubator shaker with agitation. After 3 days, all slurries and wet solids were filtered centrifugally, and analyzed by XRPD.

### Results

Starting from amorphous SCO-101, the following crystal forms were obtained depending on the solvent and crystallization technique used, c.f. Table 1.

**Table 1: Overview of different solvent systems and crystallization techniques, and resulting crystal form of SCO-101 obtained**

| **Entry** | **Form** | **Pattern** | **Solvent** | **Technique** | **Description** |
|---|---|---|---|---|---|
| 1 | I | 1 | Isopropyl acetate | Method A2 | Anhydrous non-solvate |
| 2 | | 2 | 1,4-dioxane | Method A1 | Mono-solvate |
| 3 | | 3 | 1-Propanol | Method A1 | Mono-solvate |
| 4 | V | 5 | 2-Propanol | Method A1 | Mono-solvate |
| 5 | | 10 | MEK | Method A1 | Mono-solvate |
| 6 | | 13 | 1-Butanol | Method A1 | Mono-solvate |
| 7 | | 14 | DMSO | Method A1 | Mono-solvate |
| 8 | | 16 | DMA | Method A1 | Solvate |
| 9 | | 17 | DMF | Method A1 | Solvate |
| 10 | | 22 | Ethanol | Method C | Solvate |
| 11 | | 23 | Acetone | Method C | Mono-solvate |
| 12 | III | 24 | Methanol | Method C | Anhydrous non-solvate |
| 13 | IV | 29 | DCM | Method B | Anhydrous non-solvate |
| 14 | | 36 | NMP | Method A2 | Solvate |

### Conclusions

Anhydrous, non-solvate crystal forms of SCO-101 can be prepared using the conditions of table 1 entries 1, 13, and 14 to provide forms I, III, and IV, respectively.

Further, the present example demonstrates that a wide range of solvates can be formed resulting in different crystal forms (patterns) of SCO-101. By selecting the conditions described in Table 1, the desired crystal forms can be provided.

### Example 4: Preparation of crystal form I

### Materials

SCO-101 crude was manufactured using the method described in example 1.

### Methods

SCO-101_crude (50 g, 1.0 eq., 101 mmol) was dissolved in 2-propanol (257 g) in a 1 L reactor. The reaction mixture was heated at 50 °C until a clear solution was obtained. To the solution, water (326 g) was added portion wise over a period of 30 min. After cooling the resulting slurry to 20 °C over a period of 1 h SCO-101 crude was isolated by filtration. The filter cake was washed with a mixture of 2-propanol/water (50/50% w/w, 39 g) before SCO-101 crude was re-dissolved in acetone (154 g) at 10 ± 5 °C. To the solution was then added water (195 g) over a period of 70 to 90 min. The crystallized SCO-101 was isolated and washed with a mixture of acetone/water (45:55% w/w, 180 g). The product was dried in an oven under reduced pressure at 45 °C.

### Results

The SCO-101 Form I prepared by the method above has been analyzed by XRPD (Fig. 1A), TG/DSC (Fig. 2A) and DVS (Fig. 3A, as well as submitted to competitive slurry experiments further described in Example 7.

### Conclusions

SCO-101 Form I has been shown to be non-hygroscopic and the thermodynamically most stable polymorph of SCO-101. In addition, Form I has shown superior properties when compared to the other non-solvated forms and solvated forms of SCO-101. Form I has the highest melting point, and together with the results from the competitive slurry experiments, it can be concluded that Form I is the thermodynamically most stable polymorph of SCO-101. Furthermore, DVS analysis of Form I and Form II, has shown that Form I is essentially non-hygroscopic, whereas Form II absorbs approx. 6% (w/w) at 90% RH.

### Example 5: Preparation of crystal form III (Pattern 24)

### Materials

Amorphous SCO-101 was prepared as described in Example 2.

### Methods

A slurry of amorphous SCO-101 in methanol was prepared in a scintillation vial. The vial was capped, sealed with parafilm and temperature cycled between room temperature and 40°C over 4 hour cycles in an incubator shaker.

After 24 hrs. A wet sample showed the formation of Pattern 38. The solid was recovered via centrifugation, and dried at 40°C for 24 hrs.

### Results

The solid was analyzed by XRPD and the formation of Pattern 24 was confirmed (Fig. 1C). TG/DSC analysis (Fig. 2C) confirms the formation of a non-solvate crystal form of SCO-101. The DSC analysis shows an exothermic event with an onset at 162°C. By VT-XRPD it has been confirmed that the exothermic event is caused by a conversion to Pattern 29 (Form IV).

### Conclusions

A non-solvate crystal form of SCO-101 (Form III, Pattern 24) can be obtained by temperature cycling of a slurry of amorphous SCO-101 in methanol.

### Example 6: Preparation of crystal form IV (Pattern 29)

### Materials

Form III SCO-101 was prepared as described in Example 5.

### Methods

200 mg SCO-101, Form III (Pattern 24) was placed at 40°C/75% RH in an open vial covered with tissue for 3 days.

### Results

After 3 days, the solid was analysed by XRPD and the formation of Pattern 29 (Form IV) was confirmed (Fig. 1D).

TG/DSC analysis (Fig. 2D) confirms the formation of a non-solvate crystal form of SCO-101.

### Conclusions

The non-solvate crystal form of SCO-101 (Form IV, Pattern 29) can be obtained by storage of Form III at 40°C/75% relative humidity (RH) in an open vial for 3 days.

### Example 7: Conversion of metastable crystal forms to thermodynamically stable form, Form I

### Materials

The following materials have been used for the competitive slurry experiments aiming at providing the thermodynamically stable polymorph of SCO-101:
*Non-solvates*: Form I, (example 1), Form II (example 1), Form III (example 5), and Form IV (example 6).
Solvates: Pattern 5, Pattern 22, and Pattern 23 (example 3)

### Methods

Saturated solutions of SCO-101 in acetone:water (50:50% v/v) and acetone:heptane (50:50% v/v) were prepared by stirring slurries of SCO-101 on these solvent systems for 15 min at 40°C and then filtering the slurries into pre-warmed vials using pre-warmed syringes.
For each experiment, the amounts of the relevant forms/patterns of SCO-101 listed in table 2 were added to the indicated volume of the saturated solutions to form slurries. The slurries were stirred at 40°C for 3 days. After 3 days, the solids were filtered centrifugally and analysed by XRPD.

### Results

The results of the competitive slurry experiments are listed in table 2:

**Table 2. Overview of results from competitive slurry experiments. All forms/patterns described in table 2 could successfully be converted to Form I. Pattern 5 is also described as Form V herein.**

| No | Input | | Solvent system | Form/Pattern after slurrying for 3 days |
|---|---|---|---|---|
| 1 | Form I (20 mg) | Form II (20 mg) | Acetone:water (1.5 mL | Form I |
| 2 | | | Acetone:heptane (1.5 mL) | Form I |
| 3 | Form I (10 mg) | Pattern 5 (10 mg) | Acetone:water (0.5 mL | Form I |
| 4 | | | Acetone:heptane (0.5 mL) | Form I |
| 5 | Form I (10 mg) | Pattern 22 (10 mg) | Acetone:water (0.5 mL | Form I |
| 6 | | | Acetone:heptane (0.5 mL) | Form I |
| 7 | Form I (10 mg) | Pattern 23 (10 mg) | Acetone:water (0.5 mL | Form I |
| 8 | | | Acetone:heptane (0.5 mL) | Form I |
| 9 | Form I (10 mg) | Form III (10 mg) | Acetone:water (0.5 mL | Form I |
| 10 | | | Acetone:heptane (0.5 mL) | Form I |
| 11 | Form I (10 mg) | Form IV (10 mg) | Acetone:water (0.5 mL | Form I |
| 12 | | | Acetone:heptane (0.5 mL) | Form I |

### Conclusions

The slurry experiments shows that the crystalline, non-solvated Form I of SCO-101 is the thermodynamically stable polymorph. All experiments in the tested solvent systems results in the formation of Form I.

### Example 8: Methods of analysis

### X-ray Powder Diffraction (XRPD)

XRPD analysis was carried out on a PANalytical X'pert pro with PlXcel detector (128 channels), scanning the samples between 3 and 50° 2θ. The material was gently ground to release any agglomerates and loaded onto a multi-well plate with Mylar polymer film to support the sample. The multi-well plate was then placed into the diffractometer and analysed using Cu K radiation (α₁ λ = 1.54060 Å; α₂ = 1.54443 Å; β = 1.39225 Å; α₁ : α₂ ratio = 0.5) running in transmission mode (step size 0.0130° 2θ, step time 18.87s) using 40 kV / 40 mA generator settings. Data were visualized and images generated using the HighScore Plus 4.7 desktop application (PANalytical, 2017).

### Polarised Light Microscopy (PLM)

The presence of crystallinity (birefringence) was determined using an Olympus BX53 microscope, equipped with cross-polarising lenses and a Motic camera. Images were captured using Motic Images Plus 3.0. All images were recorded using the 20 × objective, unless otherwise stated.

### Thermogravimetric Analysis/ Differential Scanning Calorimetry (TGA/DSC)

Approximately 5-10 mg of material was added into a pre-tared open aluminium pan and loaded into a TA Instruments Discovery SDT 650 Auto - Simultaneous DSC and held at room temperature. The sample was then heated at a rate of 10 °C/min from 30 °C to 400 °C during which time the change in sample weight was recorded along with the heat flow response (DSC). Nitrogen was used as the sample purge gas, at a flow rate of 200 cm³/min.

### Differential Scanning Calorimetry (DSC)

Approximately 1-5 mg of material was weighed into an aluminium DSC pan and sealed non-hermetically with an aluminium lid. The sample pan was then loaded into a TA Instruments Discovery DSC 2500 differential scanning calorimeter equipped with a RC90 cooler. The sample and reference were heated to 200 °C at a scan rate of 10 °C/min and the resulting heat flow response monitored. The sample was re-cooled to - 80 °C and then reheated again to 200 °C all at 10 °C/min. Nitrogen was used as the purge gas, at a flow rate of 50 cm³/min.

### Infrared Spectroscopy (IR)

Infrared spectroscopy was carried out on a Bruker ALPHA P spectrometer. Sufficient material was placed onto the centre of the plate of the spectrometer and the spectra were obtained using the following parameters:

| | |
|---|---|
| Resolution: | 4 cm⁻¹ |
| Background Scan Time: | 16 scans |
| Sample Scan Time: | 16 scans |
| Data Collection: | 4000 to 400 cm⁻¹ |
| Result Spectrum: | Transmittance |
| Software: | OPUS version 6 |

### Nuclear Magnetic Resonance (NMR)

NMR experiments were performed on a Bruker AVIIIHD spectrometer equipped with a PRODIGY cryoprobe operating at 500.23 MHz for protons or on a Bruker AVIIIHD spectrometer equipped with a DCH cryoprobe operating at 500.12MHz for protons. Experiments were performed in deuterated dimethyl sulfoxide and each sample was prepared to ca. 10 mM concentration.

### Dynamic Vapour Sorption (DVS)

Approximately 10-20 mg of sample was placed into a mesh vapour sorption balance pan and loaded into a DVS Intrinsic or Advantage dynamic vapour sorption balance by Surface Measurement Systems. The sample was subjected to a ramping profile from 40 - 90% relative humidity (RH) at 10% increments, maintaining the sample at each step until a stable weight had been achieved (dm/dt 0.004%, minimum step length 30 minutes, maximum step length 120 minutes) at 25°C. After completion of the sorption cycle, the sample was dried using the same procedure to 0% RH and then a second sorption cycle back to 40% RH. Two cycles were performed. The weight change during the sorption/desorption cycles were plotted, allowing for the hygroscopic nature of the sample to be determined. XRPD analysis was then carried out on any solid retained.

## Claims

1. A crystal form I of SCO-101: 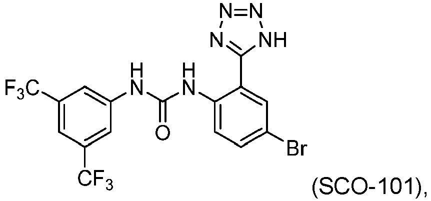 exhibiting at least peak maxima at 2 Theta angles: 19.0±0.2, 21.2±0.2, and 23.4±0.2 in an X-ray powder diffraction (XRPD) diffractogram when measured using Cu K_{α} radiation; optionally wherein the crystal form I exhibits at least peak maxima at 2 Theta angles: 13.9±0.2, 19.0±0.2, 19.9±0.2, and 21.2±0.2 in an X-ray powder diffraction (XRPD) diffractogram when measured using Cu K_{α} radiation, for example wherein the crystal form I exhibits at least peak maxima at 2 Theta angles: 12.0±0.2, 13.9±0.2, 19.0±0.2, 19.9±0.2, 20.4±0.2, 21.2±0.2, 23.2±0.2, 23.4±0.2, 26.9±0.2, and 27.4±0.2 in an X-ray powder diffraction (XRPD) diffractogram when measured using Cu K_{α} radiation.

2. The crystal form I according to claim 1, wherein the crystal form I:
a) exhibits an XRPD diffractogram according to Fig 1A when measured using Cu K_{α} radiation;
b) exhibits in differential scanning calorimetry (DSC) an onset temperature of from 218 to 226 °C using a heating rate of 10 °C per minute, such as from 219 to 224 °C, such as from 220 to 222 °C, for example 221 °e; and/or
c) I exhibits in differential scanning calorimetry (DSC) a peak temperature of from 224 to 234 °C using a heating rate of 10 °C per minute, such as from 225 to 233 °C, such as from 226 to 232 °C, such as from 227 to 231 °C, such as from 228 to 230 °C, for example 229 °C.

3. A crystal form III of SCO-101: 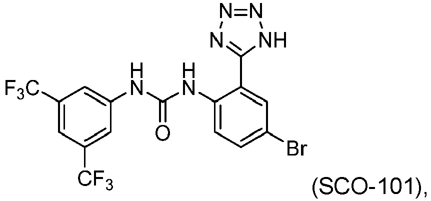 exhibiting at least peak maxima at 2 Theta angles: 11.1±0.2, 21.7±0.2, and 23.3±0.2 in an X-ray powder diffraction (XRPD) diffractogram when measured using Cu K_{α} radiation, optionally wherein the crystal form III exhibits at least peak maxima at 2 Theta angles: 11.1±0.2, 21.7±0.2, 23.3±0.2, and 26.2±0.2 in an X-ray powder diffraction (XRPD) diffractogram when measured using Cu K_{α} radiation, for example wherein the crystal form III exhibits at least peak maxima at 2 Theta angles: 11.1±0.2, 16.6±0.2, 18.0±0.2,19.2±0.2, 19.9±0.2, 21.7±0.2, 22.2±0.2, 22.5±0.2, 23.3±0.2, and 26.2±0.2 in an X-ray powder diffraction (XRPD) diffractogram when measured using Cu K_{α} radiation.

4. The crystal form III according to claim 3, wherein the crystal form III:
a) exhibits an XRPD diffractogram according to Fig 1C when measured using Cu K_{α} radiation;
b) exhibits in differential scanning calorimetry (DSC) an onset temperature of from 220 to 228 °C using a heating rate of 10 °C per minute, such as from 221 to 226 °C, such as from 222 to 224 °C, for example 223 °C; and/or
c) exhibits in differential scanning calorimetry (DSC) a peak temperature of from 225 to 235 °C using a heating rate of 10 °C per minute, such as from 226 to 234 °C, such as from 226 to 234 °C, such as from 227 to 233 °C, such as from 228 to 232 °C, such as from 229 to 231 °C, for example 230 °C.

5. A crystal form IV of SCO-101: 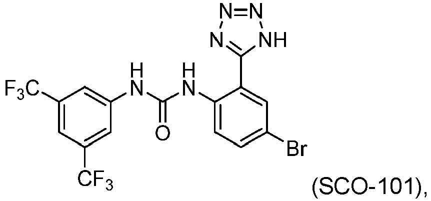 exhibiting at least peak maxima at 2 Theta angles: 22.6±0.2, 23.4±0.2, and 23.7±0.2 in an X-ray powder diffraction (XRPD) diffractogram when measured using Cu K_{α} radiation, optionally wherein the crystal form IV exhibits at least peak maxima at 2 Theta angles: 21.6±0.2, 22.6±0.2, 23.4±0.2, 23.7±0.2, and 24.1±0.2 in an X-ray powder diffraction (XRPD) diffractogram when measured using Cu K_{α} radiation, for example wherein the crystal form IV exhibits at least peak maxima at 2 Theta angles: 13.5±0.2, 20.1±0.2, 21.6±0.2, 22.6±0.2, 23.4±0.2, 23.7±0.2, 24.1±0.2, 25.6±0.2, 27.4±0.2, and 30.2±0.2 in an X-ray powder diffraction (XRPD) diffractogram when measured using Cu K_{α} radiation.

6. The crystal form IV according to claim 5, wherein the crystal form IV exhibits:
a) an XRPD diffractogram according to Fig 1D when measured using Cu K_{α} radiation;
b) exhibits in differential scanning calorimetry (DSC) an onset temperature of from 222 to 230 °C using a heating rate of 10 °C per minute, such as from 223 to 228 °C, such as from 224 to 226 °C, for example 225 °C; and/or
c) exhibits in differential scanning calorimetry (DSC) a peak temperature of from 226 to 236 °C using a heating rate of 10 °C per minute, such as from 227 to 235 °C, such as from 228 to 234 °C, such as from 229 to 233 °C, such as from 230 to 232 °C, for example 231 °C.

7. An amorphous form of SCO-101: 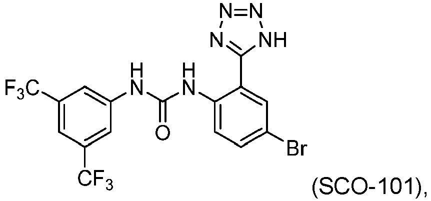 exhibiting no peak maxima at 2 Theta angles between 0 and 40 in an X-ray powder diffraction (XRPD) diffractogram when measured using Cu K_{α} radiation.

8. The amorphous form according to claim 7, wherein the amorphous form:
a) exhibits an XRPD diffractogram according to Fig 1E when measured using Cu K_{α} radiation;
b) exhibits in differential scanning calorimetry (DSC) an onset temperature of from 211 to 219 °C using a heating rate of 10 °C per minute, such as from 212 to 218 °C, such as from 213 to 217 °C, such as from 214 to 216 °C, for example 215 °C; and/or
c) exhibits in differential scanning calorimetry (DSC) a peak temperature of from 218 to 228 °C using a heating rate of 10 °C per minute, such as from 219 to 227 °C, such as from 220 to 226 °C, such as from 221 to 225 °C, such as from 222 to 224 °C, for example 223 °C.

9. A crystal form V of SCO-101 isopropanol solvate: 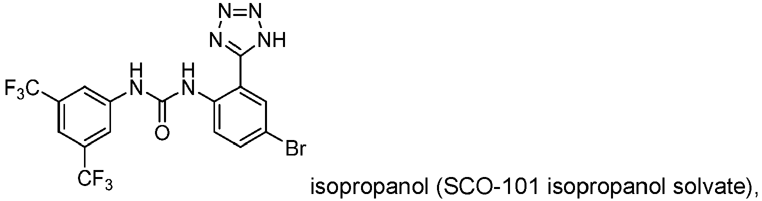 exhibiting at least peak maxima at 2 Theta angles: 9.4±0.2, 21.1 ±0.2, and 22.2±0.2 in an X-ray powder diffraction (XRPD) diffractogram when measured using Cu K_{α} radiation, optionally wherein the crystal form V exhibits at least peak maxima at 2 Theta angles: 8.2±0.2, 9.4±0.2, 21.1±0.2, and 22.2±0.2 in an X-ray powder diffraction (XRPD) diffractogram when measured using Cu K_{α} radiation; for example wherein the crystal form V exhibits at least peak maxima at 2 Theta angles: 8.2±0.2, 9.4±0.2, 10.5±0.2, 21.1±0.2, 22.2±0.2, 23.7±0.2, 24.2±0.2, 24.6±0.2, 25.5±0.2, and 28.5±0.2 in an X-ray powder diffraction (XRPD) diffractogram when measured using Cu K_{α} radiation.

10. The crystal form V according to claim 9, wherein the crystal form V:
a) exhibits an XRPD diffractogram according to Fig 1F when measured using Cu K_{α} radiation;
b) exhibits in differential scanning calorimetry (DSC) an onset temperature of from 216 to 224 °C using a heating rate of 10 °C per minute, such as from 217 to 223 °C, such as from 218 to 222 °C, such as from 219 to 221 °C, for example 220 °C; and/or
c) exhibits in differential scanning calorimetry (DSC) a peak temperature of from 224 to 234 °C using a heating rate of 10 °C per minute, such as from 225 to 233 °C, such as from 226 to 232 °C, such as from 227 to 231 °C, such as from 228 to 230 °C, for example 229 °C.

11. A process for preparing a crystal form I of SCO-101 as defined in any one of claims 1-2, the process comprising the consecutive steps of:
a) dissolving SCO-101 in one or more polar aprotic solvents selected from the group consisting of: acetone, acetonitrile, dichloromethane, dimethylformamide, dimethylpropyleneurea, dimethylsulfoxide, ethyl acetate, 2-MeTHF, and tetrahydrofuran, for example wherein the polar aprotic solvent is acetone, at a first predefined temperature from 0 to 20 °C, such as 10 ± 5 °C;
b) adding one or more polar protic solvents selected from the group consisting of: water, methanol, ethanol, isopropanol, and acetic acid, for example wherein the polar protic solvent is water to the one or more polar aprotic solvents over the course of a first predefined time period from 10 to 360 minutes, such as from 70 to 90 minutes to provide the crystal form I of SCO-101; and
c) isolating the crystal form I of SCO-101.

12. The process according to claim 11, wherein the process further comprises a prior crystallization step which is prior to step a), wherein the prior crystallization step comprises:
i) mixing a composition comprising SCO-101 and one or more impurities with 2-propanol to provide a mixture;
ii) heating the mixture to or at a second predefined temperature which is from 31 to 80 °C, such as 50 °C and which is above the first predefined temperature;
iii) adding water to the mixture over the course of a second predefined time period, such as wherein the second predefined time period is from 1 to 120 minutes, for example 30 minutes;
iv) cooling the mixture to a third predefined temperature which is optionally from 10 to 30 °C, such as 20 °C, and which is below the second predefined temperature to provide SCO-101 as a solid, optionally further isolating the solid by filtration, optionally wherein the one or more impurities are selected from the group consisting of: 4-bromo-2-(1H-1,2,3,4-tetraazol-5-yl)aniline, 3,5-bis(trifluoromethyl)-phenyl isocyanate, and toluene.

13. A process for preparing the crystal form I of SCO-101 as defined in any one of claims 1-2 from a metastable form, comprising:
a) providing a metastable form which is a crystal form or an amorphous form of SCO-101;
b) mixing the metastable form with the crystal form I of SCO-101 as defined in any one of claims 1-2 in a solvent mixture of: i) one or more polar aprotic solvents selected from the group consisting of: acetone, acetonitrile, dichloromethane, dimethylformamide, dimethylpropyleneurea, dimethylsulfoxide, ethyl acetate, 2-MeTHF, and tetrahydrofuran, and
ii) one or more polar protic solvents selected from the group consisting of: water, methanol, ethanol, isopropanol, and acetic acid, or one or more apolar solvents selected from the group consisting of: heptane, hexane, pentane, cyclohexane, toluene, and diethyl ether; for example wherein the polar aprotic solvent is acetone and the polar protic solvent is water;
c) agitating the solvent mixture at from 30 to 60 °C for at least 1 hour thereby providing the crystal form I of SCO-101;
wherein the crystal form of SCO-101 is selected from the group consisting of:
i) a crystal form II of SCO-101: 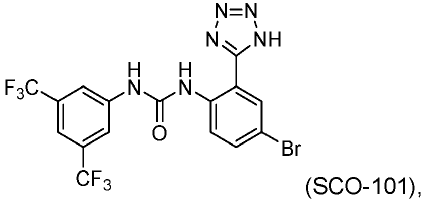 exhibiting at least peak maxima at 2 Theta angles: 18.8±0.2, 23.2±0.2, and 20.5±0.2 in an X-ray powder diffraction (XRPD) diffractogram when measured using Cu K_{α} radiation;
ii) a crystal form III of SCO-101 as defined in any one of claims 3-4;
iii) a crystal form IV of SCO-101 as defined in any one of claims 5-6; and
iv) a crystal form V of SCO-101 as defined in any one of claims 9-10, and
wherein the amorphous form of SCO-101 is as defined in any one of claims 7-8.

14. A pharmaceutical composition comprising a crystal form I of SCO-101 as defined in any one of claims 1-2; and one or more pharmaceutically acceptable adjuvants, excipients, carriers, buffers and/or diluents.

15. A crystal form I of SCO-101 as defined in any one of claims 1-2, or a pharmaceutical composition as defined in claim 14 for use in the treatment of cancer in combination with one or more anti-cancer agents; optionally wherein the one or more anti-cancer agents are selected from the group consisting of topoisomerase inhibitors, anti-hormone agents, alkylating agents, mitotic inhibitors, antimetabolites, anti-tumor antibiotics, corticosteroids, targeted anti-cancer therapy, differentiating agents and immunotherapy.

## Patentansprüche

1. Kristallform I von SCO-101: 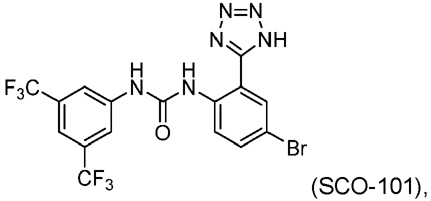 wobei sie mindestens Spitzenmaxima bei 2-Theta-Winkeln aufweisen: 19,0 ± 0,2, 21,2 ± 0,2 und 23,4 ± 0,2 in einem Röntgenpulverdiffraktogramm (XRPD), wenn sie unter Verwendung von Cu-K_{α}-Strahlung gemessen wird; wobei optional die Kristallform I mindestens Spitzenmaxima bei 2-Theta-Winkeln aufweist: 13,9 ± 0,2, 19,0 ± 0,2, 19,9 ± 0,2 und 21,2 ± 0,2 in einem Röntgenpulverdiffraktogramm (XRPD), wenn sie unter Verwendung von Cu-K_{α}-Strahlung gemessen wird, wobei beispielsweise die Kristallform I mit mindestens Spitzenmaxima Winkel 2 Theta: 12,0 ± 0,2, 13,9 ± 0,2, 19,0 ± 0,2, 19,9 ± 0,2, 20,4 ± 0,2, 21,2 ± 0,2, 23,2 ± 0,2, 23,4 ± 0,2, 26,9 ± 0,2, und 27,4 ± 0,2 in einem Röntgenpulverdiffraktogramm (XRPD), wenn sie unter Verwendung von Cu-K_{α}-Strahlung gemessen wird.

2. Kristallform I nach Anspruch 1, wobei die Kristallform I:
a) ein XRPD-Diffraktogramm in Fig. 1A, wenn sie unter Verwendung von Cu-K_{α}-Strahlung gemessen wird_{,} aufweist;
b) in der Differential Scanning Calorimetry (DSC) eine Anfangstemperatur von 218 bis 226°C bei einer Heizrate von 10°C pro Minute, beispielsweise von 219 bis 224°C, beispielsweise von 220 bis 222°C, beispielsweise von 221°C, aufweist; und/oder
c) eine maximale Temperatur von 224 bis 234°C bei einer Heizrate von 10°C pro Minute, beispielsweise von 225 bis 233°C, beispielsweise von 226 bis 232°C, beispielsweise von 227 bis 231°C, beispielsweise von 228 bis 230°C, beispielsweise von 229°C, aufweist.

3. Kristallform III von SCO-101: 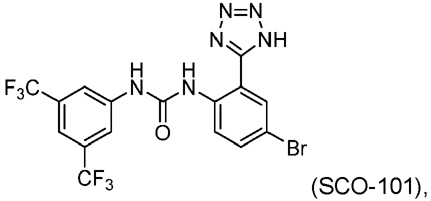 wobei sie mindestens Spitzenmaxima bei 2-Theta-Winkeln aufweisen: 11,1 ± 0,2, 21,7 ± 0,2 und 23,3 ± 0,2 in einem Röntgenpulverdiffraktogramm (XRPD), wenn sie unter Verwendung von Cu-K_{α}-Strahlung gemessen wird, wobei optional die Kristallform III mindestens Spitzenmaxima bei 2-Theta-Winkeln aufweit: 11,1 ± 0,2, 21,7 ± 0,2, 23,3 ± 0,2 und 26,2 ± 0,2 in einem Röntgenpulverdiffraktogramm (XRPD), wenn sie unter Verwendung von Cu-K_{α}-Strahlung gemessen wird, wobei beispielweise die Kristallform III mindestens Spitzenmaxima bei 2-Theta-Winkeln: 11,1 ± 0,2, 16,6 ± 0,2, 18,0 ± 0,2, 19,2 ± 0,2, 19,9 ± 0,2, 21,7 ± 0,2, 22,2 ± 0,2, 22,5 ± 0,2, 23,3 ± 0,2 und 26,2 ± 0,2 in einem Röntgenpulverdiffraktogramm (XRPD) wenn sie unter Verwendung von Cu-K_{α}-Strahlung gemessen wird, aufweist.

4. Kristallform III nach Anspruch 3, wobei die Kristallform III:
a) ein XRPD-Diffraktogramm in Fig. 1C, wenn sie unter Verwendung von Cu-K_{α}-Strahlung gemessen wird_{,} aufweist;
b) in der Differential Scanning Calorimetry (DSC) eine Anfangstemperatur von 220 bis 228°C bei einer Heizrate von 10°C pro Minute, beispielsweise von 221 bis 226°C, beispielsweise von 222 bis 224°C, beispielweise von 223°C, aufweist; und/oder
c) in der Differential Scanning Calorimetry (DSC) eine maximalen Temperatur von 225 bis 235°C bei einer Heizrate von 10°C pro Minute, beispielsweise von 226 bis 234°C, beispielsweise von 226 bis 234°C, beispielweise von 227 bis 233°C, Beispielweise von 228 bis 232°C, beispielweise von 229 bis 231°C, beispielweise von 230°C, aufweist.

5. Kristallform IV von SCO-101: 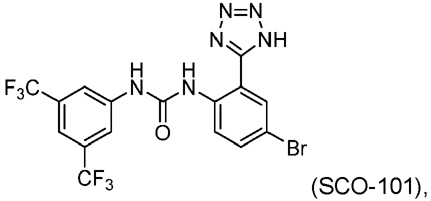 wobei sie mindestens Spitzenmaxima bei 2-Theta-Winkeln: 22,6 ± 0,2, 23,4 ± 0,2 und 23,7 ± 0,2 in einem Röntgenpulverdiffraktogramm (XRPD), wenn es unter Verwendung von Cu-K_{α}-Strahlung gemessen wird, aufweist, wobei optional die Kristallform IV mindestens Spitzenmaxima bei 2-Theta-Winkeln: 21,6 ± 0,2, 22,6 ± 0,2, 23,4 ± 0,2, 23,7 ± 0,2 und 24,1 ± 0,2 in einem Röntgenpulverdiffraktogramm (XRPD), wenn sie unter Verwendung von Cu-K_{α}-Strahlung gemessen wird, wobei beispielsweise die Kristallform IV mindestens Spitzenmaxima bei Winkeln von 2-Theta-Winkeln: 13,5 ± 0,2, 20,1 ± 0,2, 21,6 ± 0,2, 22,6 ± 0,2, 23,4 ± 0,2, 23,7 ± 0,2, 24,1 ± 0,2, 25,6 ± 0,2, 27,4 ± 0,2 und 30,2 ± 0,2 in einem Röntgenpulverdiffraktogramm (XRPD), wenn sie unter Verwendung von Cu-K_{α}-Strahlung gemessen wird, aufweist.

6. Kristallform IV nach Anspruch 5, wobei die Kristallform IV:
a) ein XRPD- Diffraktogramm in Fig. 1D, wenn sie unter Verwendung von Cu-K_{α}-Strahlung gemessen wird, aufweist;
b) in der Differential Scanning Calorimetry (DSC) eine Anfangstemperatur von 222 bis 230°C bei einer Heizrate von 10°C pro Minute, beispielsweise von 223 bis 228°C, beispielsweise von 224 bis 226°C, beispielweise von 225°C aufweist; und/oder
c) in der Differential Scanning Calorimetry (DSC) eine maximale Temperatur von 226 bis 236°C bei einer Heizrate von 10°C pro Minute, beispielsweise von 227 bis 235°C, beispielsweise von 228 bis 234°C, beispielweise von 229 bis 233°C, beispielsweise von 230 bis 232°C, beispielsweise von 231°C, aufweist.

7. Amorphe Form von SCO-101: 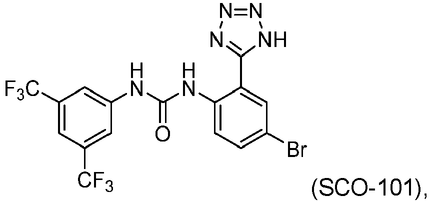 wobei sie keine Spitzenmaxima bei 2-Theta-Winkeln zwischen 0 und 40 in einem Röntgenpulverdiffraktogramm (XRPD), wenn sie unter Verwendung von Cu-K_{α}-Strahlung gemessen wird, aufweist.

8. Amorphe Form nach Anspruch 7, wobei die amorphe Form:
a) ein XRPD-Diffraktogramm in Fig. 1E, wenn sie unter Verwendung von Cu-K_{α}-Strahlung gemessen wird, aufweist;
b) in der Differential Scanning Calorimetry (DSC) eine Anfangstemperatur von 211 bis 219°C bei einer Heizrate von 10°C pro Minute, beispielsweise von 212 bis 218°C, beispielsweise von 213 bis 217°C von 214 bis 216°C, beispielweise von 215°C, aufweist; und/oder
c) in der Differential Scanning Calorimetry (DSC) eine maximale Temperatur von 218 bis 228°C bei einer Heizrate von 10°C pro Minute, beispielsweise von 219 bis 227°C, beispielsweise von 220 bis 226°C von 221 bis 225°C, beispielsweise von 222 bis 224°C, beispielsweise von 223°C, aufweist.

9. Kristallform V des Isopropanolsolvats von SCO-101: 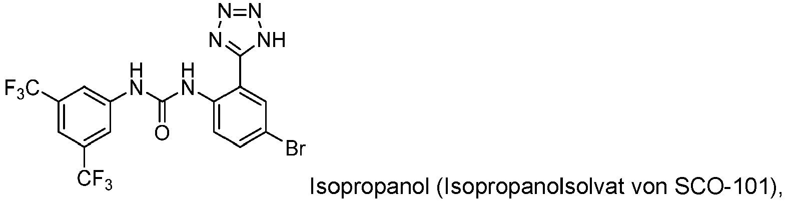 wobei sie mindestens Spitzenmaxima bei 2-Theta-Winkeln: 9,4 ± 0,2, 21,1 ± 0,2 und 22,2 ± 0,2 in einem Röntgenpulverdiffraktogramm (XRPD), wenn sie unter Verwendung von Cu-K_{α}-Strahlung gemessen wird, aufweist, wobei optional die Kristallform V mindestens Spitzenmaxima bei 2-Theta-Winkeln: 8,2 ± 0,2, 9,4 ± 0,2, 21,1 ± 0,2 und 22,2 ± 0,2 in einem Röntgenpulverdiffraktogramm (XRPD), wenn sie unter Verwendung von Cu-K_{α}-Strahlung gemessen wird, aufweist; wobei beispielweise die Kristallform V mindestens Spitzenmaxima bei 2-Theta-Winkeln: 8,2 ± 0,2, 9,4 ± 0,2, 10,5 ± 0,2, 21,1 ± 0,2, 22,2 ± 0,2, 23,7 ± 0,2, 24,2 ± 0,2, 24,6 ± 0,2, 25,5 ± 0,2 und 28,5 ± 0,2 in einem Röntgenpulverdiffraktogramm (XRPD), wenn sie unter Verwendung von Cu-K-_{α}-Strahlung gemessen wird, aufweist.

10. Kristallform V nach Anspruch 9, wobei die Kristallform V:
a) ein XRPD-Diffraktogramms in Fig. 1F, wenn sie unter Verwendung von Cu-K_{α} -Strahlung gemessen wird, aufweist;
b) in der Differential Scanning Calorimetry (DSC) eine Anfangstemperatur von 216 bis 224°C bei einer Heizrate von 10°C pro Minute, beispielsweise von 217 auf 223°C, beispielsweise von 218 auf 222°C, beispielweise von 219 bis 221°C, beispielweise von 220°C, aufweist; und/oder
c) in der Differential Scanning Calorimetry (DSC) eine maximale Temperatur von 224 bis 234°C bei einer Heizrate von 10°C pro Minute, beispielsweise von 225 bis 233°C, beispielsweise von 226 bis 232°C, beispielweise von 227 bis 231°C, beispielweise von 228 bis 230°C, beispielweise von 229°C, aufweist.

11. Verfahren zur Herstellung einer kristallform I von SCO-101 gemäß einem der Ansprüche 1 bis 2, wobei das Verfahren die folgenden aufeinanderfolgenden Schritte umfasst:
a) Auflösen von SCO-101 in einem oder mehreren polaren aprotischen Lösungsmitteln, ausgewählt aus der Gruppe, die aus Aceton, Acetonitril, Dichlormethan, Dimethylformamid, Dimethylpropylenharnstoff, Dimethylsulfoxid, Ethylacetat, 2- MeTHF und Tetrahydrofuran besteht, wobei beispielsweise das polare aprotische Lösungsmittel Aceton auf einer ersten vordefinierten Temperatur von 0 bis 20°C, beispielsweise 10 ± 5°C, ist;
b) Zugeben eines oder mehrerer polarer protischer Lösungsmittel, ausgewählt aus der Gruppe, die aus Wasser, Methanol, Ethanol, Isopropanol und Essigsäure besteht, wobei beispielsweise das polare protische Lösungsmittel Wasser ist, zu einem oder mehreren polaren aprotischen Lösungsmitteln innerhalb eines ersten vordefinierten Zeitintervalls von 10 Minuten bis 360 Minuten, beispielsweise 70 bis 90 Minuten, um die Kristallform I von SCO-101 bereitzustellen; und
c) Isolieren der Kristallform I von SCO-101.

12. Verfahren nach Anspruch 11, wobei das Verfahren weiterhin einen vorherigen Kristallisationsschritt umfasst, der vor Schritt a) liegt, wobei der vorherige Kristallisationsschritt die folgenden Schritte umfasst:
i) Mischen einer Zusammensetzung, die SCO-101 und eine oder mehrere Verunreinigungen umfasst, mit 2-Propanol, um eine Mischung bereitzustellen;
ii) Erhitzen der Mischung auf oder bei einer zweiten vordefinierten Temperatur, die 31 bis 80°C, beispielsweise 50°C, beträgt und höher als die erste vordefinierte Temperatur ist;
iii) Zugeben von Wasser zu der Mischung in einem zweiten vordefinierten Zeitintervall, wobei beispielsweise das zweite vordefinierte Zeitintervall 1 bis 120 Minuten, beispielsweise 30 Minuten, beträgt;
iv) Abkühlen der Mischung auf eine dritte vordefinierte Temperatur, die zwischen 10 und 30°C, beispielsweise 20°C, beträgt, und niedriger als die zweite vordefinierte Temperatur ist, um SCO-101 in Form eines festen Materials bereitzustellen, optional weiter Isolieren des Feststoffmaterials durch Filtration, wobei optional die eine oder mehrere Verunreinigungen aus der Gruppe, die aus 4-Brom-2-(1H-1,2,3,4-tetraazol-5-yl)anilin, 3,5-bis(Trifluormethyl)-phenylisocyanat und Toluol besteht, ausgewählt werden.

13. Verfahren zur Herstellung der Kristallform I von SCO-101 gemäß einem der Ansprüche 1 und 2 aus einer metastabilen Form, wobei das Verfahren die folgenden Schritte umfasst:
a) Bereitstellung einer metastabilen Form, die eine Kristallform oder eine amorphe Form von SCO-101 ist;
b) Mischen der metastabilen Form mit der Kristallform I von SCO-101 gemäß einem der Ansprüche 1 bis 2 in einem Lösungsmittelgemisch aus:
i) einem oder mehreren polaren aprotischen Lösungsmitteln, ausgewählt aus der Gruppe, die aus Aceton, Acetonitril, Dichlormethan, Dimethylformamid, Dimethylpropylenharnstoff, Dimethylsulfoxid, Ethylacetat, 2-MeTHF und Tetrahydrofuran, besteht, und
ii) einem oder mehreren polaren protische Lösungsmittel, ausgewählt aus der Gruppe, die aus Wasser, Methanol, Ethanol, Isopropanol und Essigsäure, besteht, oder einem oder mehreren apolaren Lösungsmittel, ausgewählt aus der Gruppe, die aus Heptan, Hexan, Pentan, Cyclohexan, Toluol und Diethylether besteht; wobei beispielweise das aprotische polare Lösungsmittel Aceton und das protische polare Lösungsmittel Wasser ist;
c) Rühren der Lösungsmittelmischung bei 30-60°C für mindestens 1 Stunde, was zur Kristallform I von SCO-101 führt; wobei die kristalline Form von SCO-101 aus der Gruppe ausgewählt wird, die aus:
i) einer Kristallform II von SCO-101: 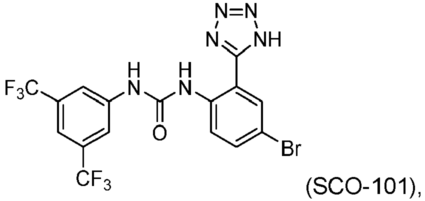 wobei sie mindestens Spitzenmaxima bei 2-Theta-Winkeln: 18,8 ± 0,2, 23,2 ± 0,2 und 20,5 ± 0,2 in einem Röntgenpulverdiffraktogramm (XRPD), wenn sie unter Verwendung von Cu-K_{α}-Strahlung gemessen wird, aufweist;
ii) einer Kristallform III von SCO-101, wie in einem der Ansprüche 3 und 4 definiert;
iii) einee Kristallform IV von SCO-101, wie in einem der Ansprüche 5 bis 6 definiert; und
iv) einer Kristallform V von SCO-101, wie in einem der Ansprüche 9 bis 10 definiert, und
wobei die amorphe Form von SCO-101, wie in einem der Ansprüche 7 bis 8 definiert ist, besteht.

14. Pharmazeutische Zusammensetzung, umfassend eine Kristallform I von SCO-101 wie in einem der Ansprüche 1 bis 2 definiert; und ein oder mehrere pharmazeutisch verträgliche Adjuvantien, Hilfsstoffe, Träger, Puffer und/oder Verdünnungsmittel.

15. Kristallform I von SCO-101 wie in einem der Ansprüche 1 bis 2 definiert oder pharmazeutische Zusammensetzung wie in Anspruch 14 definiert, zur Verwendung bei der Behandlung von Krebs in Kombination mit einem oder mehreren Antikrebsmitteln; wobei optional das eine oder die mehreren Antikrebsmittel aus der Gruppe ausgewählt, die aus Topoisomeraseinhibitoren, Antihormonmitteln, Alkylierungsmitteln, Mitoseinhibitoren, Antimetabolitinhibitoren, Antitumorantibiotika, Kortikosteroiden, gezielter Antikrebstherapie, Differenzierungsmitteln und Immuntherapie besteht.

## Revendications

1. Forme cristalline I de SCO-101 : 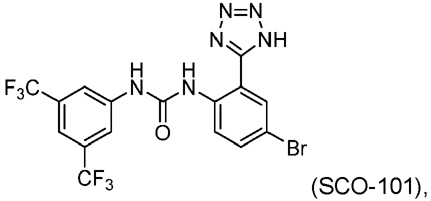 présentant au moins des maxima de pic à des angles 2 théta : 19,0 ± 0,2, 21,2 ± 0,2 et 23,4 ± 0,2 dans un diffractogramme de diffraction des rayons X sur poudre (XRPD) lorsqu'elle est mesurée à l'aide d'un rayonnement Cu K_{α} ; facultativement, la forme cristalline I présentant au moins des maxima de pic à des angles 2 théta : 13,9 ± 0,2, 19,0 ± 0,2, 19,9 ± 0,2 et 21,2 ± 0,2 dans un diffractogramme de diffraction des rayons X sur poudre (XRPD) lorsqu'elle est mesurée à l'aide d'un rayonnement Cu K_{α}, par exemple la forme cristalline I présentant au moins des maxima de pic 2 angles 2 théta : 12,0 ± 0,2, 13,9 ± 0,2, 19,0 ± 0,2, 19,9 ± 0,2, 20,4 ± 0,2, 21,2 ± 0,2, 23,2 ± 0,2, 23,4 ± 0,2, 26,9 ± 0,2 et 27,4 ± 0,2 dans un diffractogramme de diffraction des rayons X sur poudre (XRPD) lorsqu'elle est mesurée à l'aide d'un rayonnement Cu K_{α}.

2. Forme cristalline I selon la revendication 1, la forme cristalline I :
a) présentant un diffractogramme XRPD selon la figure 1A lorsqu'elle est mesurée à l'aide d'un rayonnement Cu K_{α} ;
b) présentant en calorimétrie différentielle à balayage (DSC) une température initiale de 218 à 226 °C à une vitesse de chauffage de 10 °C par minute, par exemple de 219 à 224 °C, par exemple de 220 à 222 °C, par exemple 221 °C ; et/ou
c) présentant en calorimétrie différentielle à balayage (DSC) une température maximale de 224 à 234 °C à une vitesse de chauffage de 10 °C par minute, par exemple de 225 à 233 °C, par exemple de 226 à 232 °C, par exemple de 227 à 231 °C, par eemple de 228 à 230 °C, par exemple de 229 °C.

3. Forme cristalline III de SCO-101 : 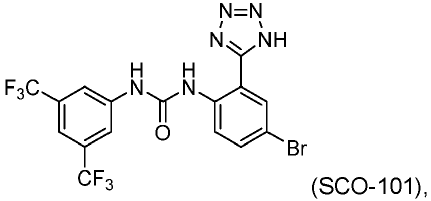 présentant au moins des maxima de pic à des angles 2 thêta : 11,1 ± 0,2, 21,7 ± 0,2 et 23,3 ± 0,2 dans un diffractogramme de diffraction des rayons X sur poudre (XRPD) lorsqu'elle est mesurée à l'aide d'un rayonnement Cu K_{α}, facultativement la forme cristalline III présentant au moins des maxima de pic à des angles 2 théta : 11,1 ± 0,2, 21,7 ± 0,2, 23,3 ± 0,2 et 26,2 ± 0,2 dans un diffractogramme de diffraction des rayons X sur poudre (XRPD) lorsqu'elle est mesurée à l'aide d'un rayonnement Cu K_{α}, par exemple la forme cristalline III présentant au moins des maxima de pics à des angles 2 thêta : 11,1 ± 0,2, 16,6 ± 0,2, 18,0 ± 0,2, 19,2 ± 0,2, 19,9 ± 0,2, 21,7 ± 0,2, 22,2 ± 0,2, 22,5 ± 0,2, 23,3 ± 0,2 et 26,2 ± 0,2 dans un diffractogramme de diffraction des rayons X sur poudre (XRPD) lorsqu'elle est mesurée à l'aide du rayonnement Cu K_{α}.

4. Forme cristalline III selon la revendication 3, la forme cristalline III :
a) présentant un diffractogramme XRPD selon la figure 1C lorsqu'elle est mesurée à l'aide d'un rayonnement Cu K_{α} ;
b) présentant en calorimétrie différentielle à balayage (DSC) une température initiale de 220 à 228 °C à une vitesse de chauffage de 10 °C par minute, par exemple de 221 à 226 °C, par exemple de 222 à 224 °C, par exemple 223 °C ; et/ou
c) présentant en calorimétrie différentielle à balayage (DSC) une température maximale de 225 à 235 °C à une vitesse de chauffage de 10 °C par minute, par exemple de 226 à 234 °C, par exemple de 226 à 234 °C, par exemple de 227 à 233 °C, par exemple de 228 à 232 °C, par exemple de 229 à 231 °C, par exemple 230 °C.

5. Forme cristalline IV de SCO-101 : 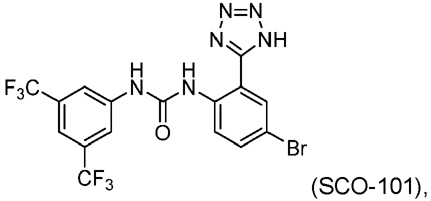 présentant au moins des maxima de pic à des angles 2 thêta : 22,6 ± 0,2, 23,4 ± 0,2 et 23,7 ± 0,2 dans un diffractogramme de diffraction des rayons X sur poudre (XRPD) lorsqu'elle est mesurée à l'aide d'un rayonnement Cu K_{α}, facultativement la forme cristalline IV présentant au moins maxima de pic à angles 2 théta : 21,6 ± 0,2, 22,6 ± 0,2, 23,4 ± 0,2, 23,7 ± 0,2 et 24,1 ± 0,2 dans un diffractogramme de diffraction des rayons X sur poudre (XRPD) lorsqu'elle est mesurée à l'aide d'un rayonnement Cu K_{α}, par exemple la forme cristalline IV présentant au moins des maxima de pic à angles 2 théta : 13,5 ± 0,2, 20,1 ± 0,2, 21,6 ± 0,2, 22,6 ± 0,2, 23,4 ± 0,2, 23,7 ± 0,2, 24,1 ± 0,2, 25,6 ± 0,2, 27,4 ± 0,2 et 30,2 ± 0,2 dans un diffractogramme de diffraction des rayons X sur poudre (XRPD) lorsqu'elle est mesurée à l'aide du rayonnement Cu K_{α}.

6. Forme cristalline IV selon la revendication 5, la forme cristalline IV présentant :
a) un diffractogramme XRPD selon la figure 1D lorsqu'elle est mesurée à l'aide d'un rayonnement Cu K_{α} ;
b) présentant en calorimétrie différentielle à balayage (DSC) une température initiale de 2 à 22 °C à une vitesse de chauffage de 10 °C par minute, par exemple de 223 à 228 °C, par exemple de 224 à 226 °C, par exemple 225 °C ; et/ou
c) présentant en calorimétrie différentielle à balayage (DSC) une température maximale de 226 à 236 °C à une vitesse de chauffage de 10 °C par minute, par exemple de 227 à 235 °C, par exemple de 228 à 234 °C, par exemple de 229 à 233 °C, par eemple de 230 à 232 °C, par exemple 231 °C.

7. Forme amorphe de SCO-101 : 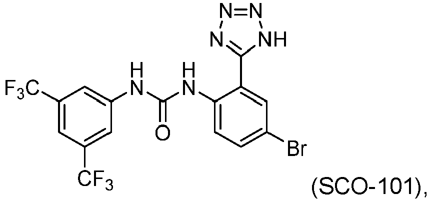 ne présentant aucun maximal de pic à des angles 2 thêta compris entre 0 et 40 dans un diffractogramme de diffraction des rayons X sur poudre (XRPD) lorsqu'elle est mesurée à l'aide du rayonnement Cu K_{α}.

8. Forme amorphe selon la revendication 7, la forme amorphe :
a) présentant un diffractogramme XRPD selon la figure 1E lorsqu'elle est mesurée à l'aide d'un rayonnement Cu K_{α} ;
b) présentant en calorimétrie différentielle à balayage (DSC) une température initiale de 211 à 219 °C à une vitesse de chauffage de 10 °C par minute, par exemple de 212 à 218 °C, par exemple de 213 à 217 °C, par exemple de 214 à 216 °C, par exemple 215 °C ; et/ou
c) présentant en calorimétrie différentielle à balayage (DSC) une température maximale de 218 à 228 °C à une vitesse de chauffage de 10 °C par minute, par exemple de 219 à 227 °C, par exemple de 220 à 226 °C, par exemple de 221 à 225 °C, par exemple de 222 à 224 °C, par exemple 223 °C.

9. Forme cristalline V du solvate d'isopropanol de SCO-101 : 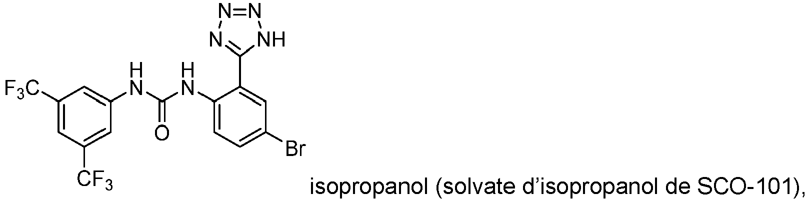 présentant au moins des maxima de pic à des angles 2 thêta : 9,4 ± 0,2, 21,1 ± 0,2 et 22,2 ± 0,2 dans un diffractogramme de diffraction des rayons X sur poudre (XRPD) lorsqu'elle est mesurée à l'aide d'un rayonnement Cu K_{α}, facultativement la forme cristalline V présentant au moins des maxima de pic à des angles 2 théta : 8,2 ± 0,2, 9,4 ± 0,2, 21,1 ± 0,2 et 22,2 ± 0,2 dans un diffractogramme de diffraction des rayons X sur poudre (XRPD) lorsqu'elle est mesurée à l'aide d'un rayonnement Cu K_{α} ; par exemple, la forme cristalline V présentant au moins des maxima de pic à des angles 2 thêta : 8,2 ± 0,2, 9,4 ± 0,2, 10,5 ± 0,2, 21,1 ± 0,2, 22,2 ± 0,2, 23,7 ± 0,2, 24,2 ± 0,2, 24,6 ± 0,2, 25,5 ± 0,2 et 28,5 ±0,2 dans un diffractogramme de diffraction des rayons X sur poudre (XRPD) lorsqu'elle est mesurée à l'aide d'un rayonnement Cu K_{α}.

10. Forme cristalline V selon la revendication 9, la forme cristalline V :
a) présentant un diffractogramme XRPD selon la figure 1F lorsqu'elle est mesurée à l'aide d'un rayonnement Cu K_{α} ;
b) présentant en calorimétrie différentielle à balayage (DSC) une température initiale de 216 à 224 °C à une vitesse de chauffage de 10 °C par minute, par exemple de 217 à 223 °C, par exemple de 218 à 222 °C, par exemple de 219 à 221 °C, par exemple 220 °C ; et/ou
c) présentant en calorimétrie différentielle à balayage (DSC) une température maximale de 224 à 234 °C à une vitesse de chauffage de 10 °C par minute, par exemple de 225 à 233 °C, par exemple de 226 à 232 °C C, par exemple de 227 à 231 °C, par exemple de 228 à 230 °C, par exemple 229 °C.

11. Procédé de préparation d'une forme cristalline I de SCO-101 telle que définie dans l'une quelconque des revendications 1 à 2, le procédé comprenant les étapes consécutives suivantes :
a) dissoudre du SCO-101 dans un ou plusieurs solvants aprotiques polaires choisis dans le groupe comprenant : l'acétone, l'acétonitrile, le dichlorométhane, le diméthylformamide, la diméthylpropylèneurée, le diméthylsulfoxyde, l'acétate d'éthyle, le 2-MeTHF et le tétrahydrofurane, par exemple le solvant aprotique polaire étant l'acétone, à une première température prédéfinie de 0 à 20 °C, par exemple 10 ± 5 °C ;
b) ajouter un ou plusieurs solvants protiques polaires sélectionnés dans le groupe comprenant : l'eau, le méthanol, l'éthanol, l'isopropanol et l'acide acétique, par exemple le solvant protique polaire étant l'eau, à un ou plusieurs des solvants aprotiques polaires dans un premier intervalle de temps prédéfini de 10 à 360 minutes, par exemple de 70 à 90 minutes pour fournir la forme cristalline I de SCO-101 ; et
c) isoler la forme cristalline I de SCO-101.

12. Procédé selon la revendication 11, le procédé comprenant en outre une étape de cristallisation préalable qui est antérieure à l'étape a), l'étape de cristallisation préalable comprenant les étapes suivantes :
i) mélanger une composition comprenant du SCO-101 et une ou plusieurs impuretés à du 2-propanol pour fournir un mélange ; ii) chauffer le mélange jusqu'à ou à une deuxième température prédéfinie qui est de 31 à 80 °C, par exemple 50 °C et qui est supérieure à la première température prédéfinie ;
iii) ajouter de l'eau au mélange dans un deuxième intervalle de temps prédéfini, par exemple le deuxième intervalle de temps prédéfini étant de 1 à 120 minutes, par exemple 30 minutes ;
iv) refroidir le mélange à une troisième température prédéfinie qui est facultativement comprise entre 10 et 30 °C, par exemple 20 °C, et qui est inférieure à la deuxième température prédéfinie pour fournir du SCO-101 sous la forme d'une matière solide, éventuellement isoler davantage la matière solide par filtration, éventuellement la ou les impuretés étant choisies dans le groupe comprenant: la 4-bromo-2-(1H-1,2,3,4-tétraazol-5-yl)aniline, le 3,5-bis(trifluorométhyl)-isocyanate de phényle et le toluène.

13. Procédé de préparation de la forme cristalline I de SCO-101 telle que définie dans l'une quelconque des revendications 1 et 2 à partir d'une forme métastable, ledit procédé comprenant les étapes suivantes :
a) fournir une forme métastable qui est une forme cristalline ou une forme amorphe de SCO-101 ;
b) mélanger la forme métastable à la forme cristalline I de SCO-101 telle que définie dans l'une quelconque des revendications 1 à 2 dans un mélange de solvants de : i) un ou plusieurs solvants aprotiques polaires choisis dans le groupe comprenant : l'acétone, l'acétonitrile, le dichlorométhane, le diméthylformamide, la diméthylpropylèneurée, le diméthylsulfoxyde, l'acétate d'éthyle, le 2-MeTHF et le tétrahydrofuranne, et ii) un ou plusieurs solvants protiques polaires choisis dans le groupe comprenant : l'eau, le méthanol, l'éthanol, l'isopropanol et l'acide acétique, ou un ou plusieurs des solvants apolaires choisis dans le groupe comprenant : l'heptane, l'hexane, le pentane, le cyclohexane, le toluène et l'éther diéthylique ; par exemple, le solvant polaire aprotique étant l'acétone et le solvant polaire protique étant l'eau ;
c) agiter le mélange de solvants entre 30 et 60 °C pendant au moins 1 heure, ce qui donne la forme cristalline I de SCO-101 ; la forme cristalline de SCO-101 étant choisie dans le groupe comprenant :
i) une forme cristalline Il de SCO-101 : 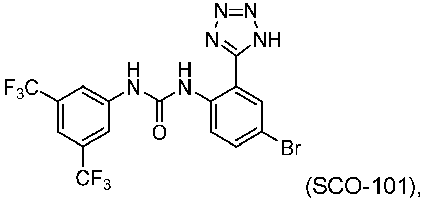 présentant au moins des maxima de pics à des angles 2 thêta : 18,8 ± 0,2, 23,2 ± 0,2 et 20,5 ± 0,2 dans un diffractogramme de diffraction des rayons X sur poudre (XRPD) lorsqu'elle est mesurée à l'aide du rayonnement Cu K_{α} ;
ii) une forme cristalline III de SCO-101 telle que définie dans l'une quelconque des revendications 3 et 4 ;
iii) une forme cristalline IV de SCO-101 telle que définie dans l'une quelconque des revendications 5 à 6 ; et
iv) une forme cristalline V de SCO-101 telle que définie dans l'une quelconque des revendications 9 à 10, et
la forme amorphe de SCO-101 étant telle que définie dans l'une quelconque des revendications 7 à 8.

14. Composition pharmaceutique comprenant une forme cristalline I de SCO-101 telle que définie dans l'une quelconque des revendications 1 à 2 ; et un ou plusieurs adjuvants, excipients, supports, tampons et/ou diluants pharmaceutiquement acceptables.

15. Forme cristalline I de SCO-101 telle que définie dans l'une quelconque des revendications 1 à 2, ou composition pharmaceutique telle que définie dans la revendication 14 destinée à ête utilisée dans le traitement de cancer en combinaison avec un ou plusieurs agents anticancéreux ; éventuellement, les un ou plusieurs agents anticancéreux étant choisis dans le groupe comprenant les inhibiteurs de la topoisomérase, les agents antihormonaux, les agents alkylants, les inhibiteurs mitotiques, d'antimétabolites, les antibiotiques antitumoraux, les corticostéroïdes, la thérapie anticancéreuse ciblée, les agents de différenciation et d'immunothérapie.
